# EUROPEAN PATENT SPECIFICATION

(11) **EP 4 208 546 B1**
(45) Date of publication and mention of the grant of the patent: **21.05.2025**
(21) Application number: 21778693.8
(22) Date of filing: 02.09.2021
(51) Int. Cl.: C12N 9/90, C12N 15/52, C12P 17/04, C12P 5/00

(54) **SQUALENE HOPENE CYCLASE DERIVATIVES AND USE THEREOF FOR PRODUCING AMBROX**
SQUALEN-HOPFENCYCLASE-DERIVATE UND IHRE VERWENDUNG ZUR HERSTELLUNG VON AMBROX
DÉRIVÉS DE SQUALÈNE HOPÈNE CYCLASE ET LEUR UTILISATION POUR LA PRODUCTION D'AMBROXAN

(30) Priority: 02.09.2020 US 202063073633 P
(43) Date of publication of application: 12.07.2023
(60) Divisional application: 25170153.8
(73) Proprietor: International Flavors & Fragrances Inc., Union Beach, New Jersey 07735 (US)
(72) Inventor: BANNON, Lyndsey Jane, Belfast, BT60 0ND (IE); DOURADO, Daniel Fernando Andrade Ribeiro, 4480-662 Vila do Conde (PT); LIPSCOMB, David Andrew, Magherafelt, BT45 5EZ (IE); MCINTYRE, Peter Brian Alexander, Belfast, BT8 7DA (IE); MIX, Stefan, Belfast, BT6 9FP (IE); MOODY, Thomas Shaw, Ballymena, BT42 1GL (IE); QUINN, Derek, Newtownards, BT23 5XJ (IE); JONES, Paul Daniel, Aberdeen, New Jersey 07747 (US); NARULA, Anubhav P.S., Hazlet, New Jersey 07730 (US)
(74) Representative: International N&H EMEA
(86) International application number: PCT/US2021/071351
(87) International publication number: WO 2022/051761

(56) References cited:
- WO-A1-2016/170099
- WO-A1-2016/170099
- WO-A1-2018/157021
- WO-A1-2021/110858
- ERIC EICHHORN ET AL: "Biocatalytic Process for (-)-Ambrox Production Using Squalene Hopene Cyclase", ADVANCED SYNTHESIS AND CATALYSIS, JOHN WILEY & SONS, INC, HOBOKEN, USA, vol. 360, no. 12, 2 May 2018 (2018-05-02), pages 2339 - 2351, XP072358471, ISSN: 1615-4150, DOI: 10.1002/ADSC.201800132
- ERIC EICHHORN ET AL: "Biocatalytic Process for (-)-Ambrox Production Using Squalene Hopene Cyclase", ADVANCED SYNTHESIS AND CATALYSIS, JOHN WILEY & SONS, INC, HOBOKEN, USA, vol. 360, no. 12, 2 May 2018 (2018-05-02), pages 2339 - 2351, XP072358471, ISSN: 1615-4150, DOI: 10.1002/ADSC.201800132

## Description

### Background

Compounds with the dodecahydronaphtho[2,1-b]furan skeleton are of great economic importance as aroma chemicals. Among these, (3aR,5aS,9aS,9bR)-dodecahydro-3a,6,6,9a-tetramethylnaphtho [2,1-b]furan), known as ambrox, is of particular importance for providing base notes of perfume compositions. Originally obtained from sperm whales' ambergris, synthetic methods have been developed for the production of ambrox. In one approach, sclareol, a constituent of clary sage (*Salvia sclarea*), is used as a starting material. Oxidative degradation of sclareol with, e.g., chromic acid, permanganate, H₂O₂ or ozone provides sclareolide, which is subsequently reduced, *e.g.,* using LiAlH₄ or NaBH₄ to give ambrox-1,4-diol. Alternatively, sclareolide can be prepared from sclareol by means of a biotransformation using *Hyphozyma roseoniger* (EP 0204009). Finally, ambra diol or tetranor labdane diol is cyclized in a series of chemical processes to give compound ambrox ((-)-2). The preparation of the racemate of ambrox, rac-2, has been accomplished, inter alia, via homofarnesylic acid and 4-(2,6,6-trimethylcyclohex-1-enyl)butan-2-one.

In another approach, ambrox is biocatalytically prepared using squalene hopene cyclase (SHC; Scheme 1) (Neumann, et al. (1986) Biol. Chem. Hoppe Seyler 367:723).

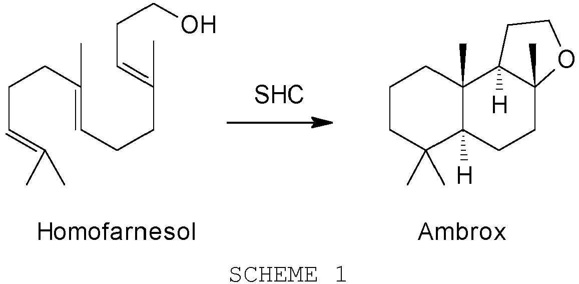

While SHC naturally catalyzes the cyclization of squalene to hopane, catalysis of ambrox is a secondary reaction with a specific activity of 0.02 mU/mg protein. SHC from *Alicyclobacillus acidocaldarius* (formerly *Bacillus acidocaldarius*) *, Zymomonas mobilis* and *Bradyrhizobium japonicum* have been purified and characterized in terms of their natural (e.g., squalene) and non-natural substrates (e.g., homofarnesol and citral). See, for example, WO 2010/139719, WO 2012/066059, JP 2009060799, and Seitz, et al. (2012) J. Molecular Catalysis B: Enzymatic 84:72-77). In addition, WO 2016/170099 describes SHC mutants with improved rates of conversion of E,E-homofarnesol to ambrox. WO2018/157021 relates to Squalene Hopene Cyclase and its use thereof for producing ambroxan.

### Summary of the Invention

This invention provides a recombinant vector as defined in claim 1. A recombinant host cell is also provided as defined in claim 2 as is a method for producing ambrox as defined in claim 4. This invention further provides a recombinant SHC polypeptide as defined in claim 3. The invention is defined by the claims and any other aspects or embodiments set forth herein not falling within the scope of the claims are for information only.

### Brief Description of the Drawings

FIG. 1A to FIG. 1C provide an amino acid sequence comparison of *Gluconobacter morbifer* Squalene Hopene Cyclase (GmSHC) with SHC enzymes from *Z. mobilis* (ZmSHC), *Bradyrhizobium sp.* (BspSHC), *Rhodopseudomonas palustris* (RpSHC), *Streptomyces coelicolor* (ScSHC), *Burkholderia ambifaria* (BamSHC), *Bacillus anthracis* (BanSHC) and A. *acidocaldarius* (AaSHC). Underlined residues represent the core sequence Gln-Xaa-Xaa-Xaa-Gly-Xaa-Trp (SEQ ID NO:3) and bolded residues represent the Asp-Xaa-Asp-Asp-Thr-Ala (SEQ ID NO:4) active site motif.
FIG. 2 shows the % peak area of ambrox produced by mutant GmSHC enzymes following incubation at 37°C for 6 and 20 hours with 15 mg/mL homofarnesol. Dashed line shows the production of ambrox by wild-type SHC.
FIG. 3 shows the % area ambrox product peak by mutant GmSHC enzymes following incubation at 37°C for 6 and 20 hours with 50 mg/mL homofarnesol at 25% enzyme loading.
FIG. 4 shows the % area ambrox product peak by mutant GmSHC enzymes following incubation at 37°C for 18 hours with 50 mg/mL homofarnesol at 5% enzyme loading.

### Detailed Description of the Invention

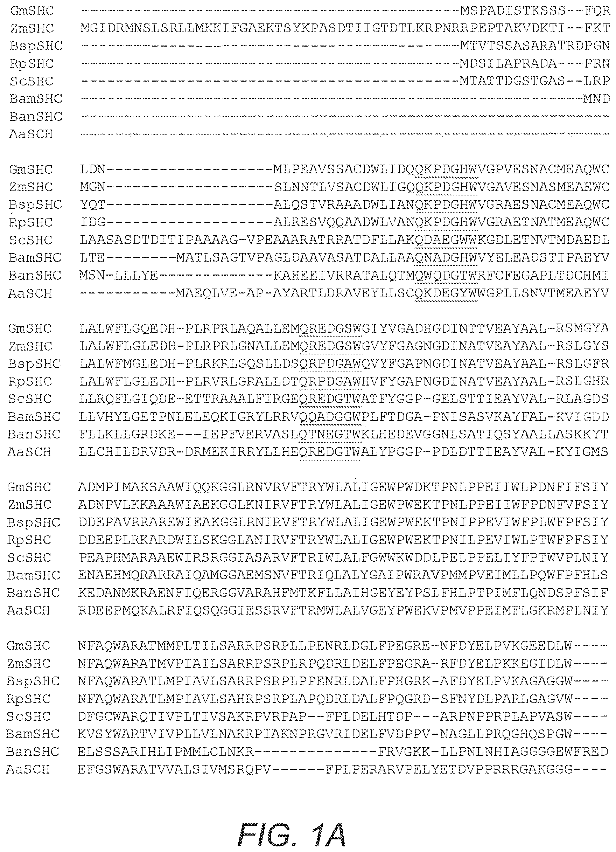

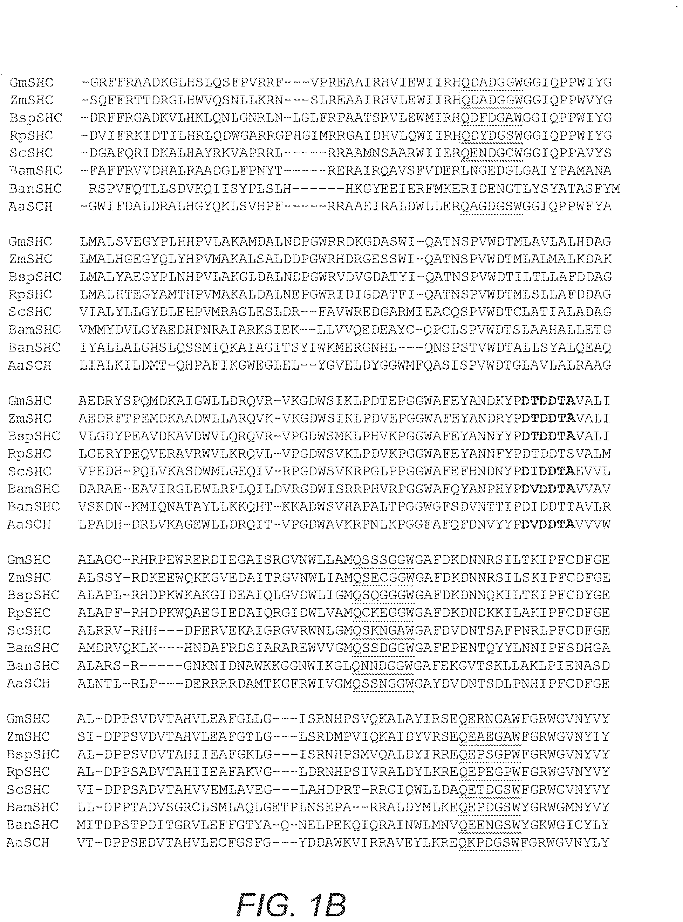

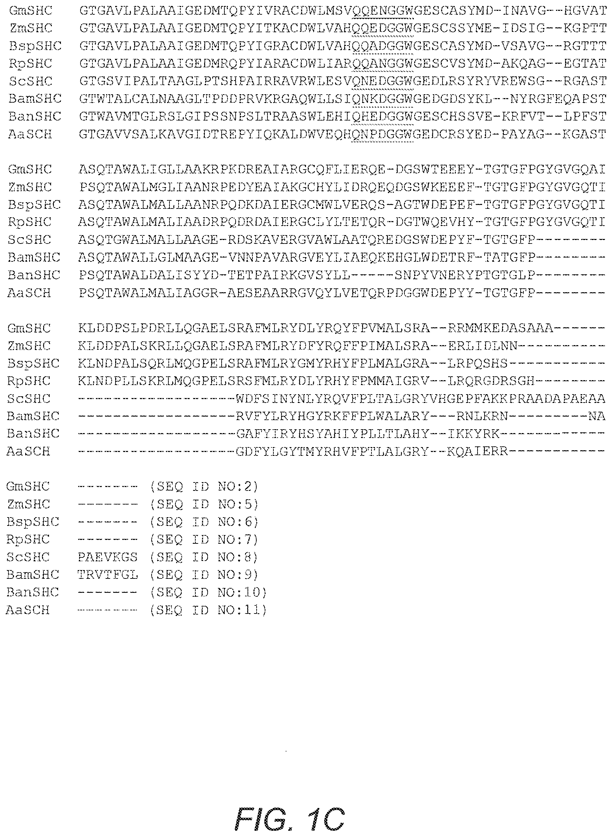

This invention provides variants of a Squalene Hopene Cyclase (SHC), or more preferably a homofarnesol-ambrox cyclase (HAC), isolated from *Gluconobacter morbifer* and method for using the variant G. *morbifer* SHC (GmSHC) to biocatalytically convert homofarnesol to ambrox. The nucleotide sequence of wild-type GmSHC is provided in SEQ ID NO:1. The amino acid sequence of wild-type GmSHC (SEQ ID NO:2) is available under GENBANK Accession Nos. WP_040507485 and EHH69691. An alignment of the GmSHC amino acid sequence with SHC amino acid sequences from *Z. mobilis, Bradyrhizobium sp., R. palustris, S. coelicolor, B. ambifaria, B. anthracis* and *A. acidocaldarius* (FIG. 1A to FIG. 1C) indicates amino acid sequence identities ranging between 37% and 76% (Table 1).

**TABLE 1**

| Source organism | Accession No. | GmSHC Identity |
|---|---|---|
| *Z. mobilis* | Q5NM88 | 76% |
| *Bradyrhizobium sp.* | ABEBP6 | 72% |
| *B. ambifaria* | Q0B5S3 | 37% |
| *B. anthracis* | A0A0E0W268 | 48% |
| *R. palustris* | WP 011665849 | 66% |
| *S. coelicolor* | Q9X7V9 | 45% |
| *A. acidocaldarius* | P33247 | 44% |

GmSHC contains the core sequence Gln-Xaa-Xaa-Xaa-Gly-Xaa-Trp (SEQ ID NO:3) (Reipen, et al. (1995) Microbiology 141:155-161), as well as the Asp-Xaa-Asp-Asp-Thr-Ala (SEQ ID NO:4) motif, which correlates with the SHC active site (Wendt, et al. (1997) Science 277:1811-5). See FIG. 1A to FIG. 1C. The data presented herein demonstrate that variants or derivatives of the GmSHC enzyme, when expressed in a heterologous host cell, *e.g., E. coli,* can readily convert homofarnesol to ambrox. Therefore, the variant or derivative GmSHC enzymes disclosed herein are of use in a method for preparing ambrox using homofarnesol as a feedstock or starting material.

As used herein, the term "ambrox" refers to (3aR, 5aS, 9aS, 9bR)-dodecahydro-3a,6,6,9a-tetramethylnaphtho [2,1-b]furan), which is known commercially as AMBROX (Firmenich), Ambroxan (Henkel) AMBROFIX (Givaudan), AMBERLYN (Quest), CETALOX Laevo (Firmenich), AMBERMOR (International Flavors and Fragrances, Aromor) and/or Norambrenolide Ether (Pacific). The desirable sensory benefits of ambrox come from the (-) stereoisomer rather than the (+) enantiomer. The odor of the (-) stereoisomer is described as musk-like, woody, warm or ambery whereas the (+) enantiomer has a relatively weak odor note. Thus, materials enriched with (-)-ambrox are one feature of this invention.

As described herein, (-)-ambrox can be synthesized from homofarnesol (Scheme 1). There are four known isomers of homofarnesol, the (3Z,7Z, *i.e.,* ZZ), (3E,7Z, *i.e.,* EZ), (3Z,7E, *i.e.,* ZE) and (3E,7E, *i.e.,* EE) isomers. According to Neumann, et al. ((1986) Biol. Chem. Hoppe Seyler 367:723), (-)-ambrox is primarily obtained from EE homofarnesol. US 2012/0135477 indicates that the *Z. mobilis* SHC enzyme can convert ZE homofarnesol to (-)-ambrox. However, Schaefer ((2011) Chemie Unserer Zeit 45:374-388) indicates that ZE homofarnesol is only converted to 9b-epi-ambrox and not to (-)-ambrox. Accordingly, the homofarnesol feedstock/starting material of this invention is a single isomer or is a mixture of two or more isomers of homofarnesol. In some embodiments, the homofarnesol starting material is a mixture of the four isomers EE:EZ:ZZ:ZE. In other embodiments, the homofarnesol starting material is a mixture of ZE:EE, ZE:EZ or EE:EZ. In embodiments including the use of a mixture of EE:EZ, preferably the weight ratio of EE:EZ is in the range of 99:1 to about 50:50. More particularly, the homofarnesol starting material has an EE:EZ weight ratio of 80:20 or 70:30. In particular embodiments, the homofarnesol starting material has >90 (3E,7E) homofarnesol. An exemplary EE:EZ stereoisomeric mixture of homofarnesol has the CAS number of 35826-67-6.

Preferably, the starting material used in the preparation of (-)-ambrox is stereoisomerically pure (3E,7E) homofarnesol (EEH). Methods for preparing EEH are known in the art and described, *e.g.,* by Dodd, et al. (1992) J. Org. Chem. 57:2794; Barrero, et al. (1996) J. Org. Chem. 61:2215; Kocienski et al. (1989) J. Org. Chem. 54:1215; WO 92/06063 and US 9,493,385.

As used herein, "GmSHC" refers to the Squalene Hopene Cyclase isolated from *Gluconobacter morbifer.* In particular, when not modified by "mutant" or "derivative," "GmSHC" refers to a wild-type protein having the amino acid sequence according to SEQ ID NO:2. By comparison, "mutant GmSHC," "variant GmSHC," "GmSHC mutant," "GmSHC variant," or "GmSHC derivative" refers to a modified or variant amino acid sequence which is altered compared to the amino acid sequence of the reference (or wild-type) GmSHC sequence according to SEQ ID NO:2. In one embodiment, a GmSHC derivative has at least one alteration that modifies (e.g., increases) the activity of the enzyme for its substrate (*e.g.,* homofarnesol, in particular EEH). In another embodiment, a GmSHC derivative has at least one alteration that modifies the stability, localization, and/or expression of the enzyme in a heterologous host cell.

As used herein, the term "amino acid alteration" means an insertion of one or more amino acid residues, a deletion of one or more amino acid residues or a substitution (which may be conservative, non-conservative or synonymous) of one or more amino acid residues relative to the amino acid sequence of a reference amino acid sequence (such as, for example, the wild-type amino acid sequence of SEQ ID NO:2). The amino acid alteration can be easily identified by a comparison of the amino acid sequences of the GmSHC derivative amino acid sequence with the amino acid sequence of the reference or wild-type GmSHC.

Conservative amino acid substitutions may be made, for instance, on the basis of similarity in polarity, charge, size, solubility, hydrophobicity, hydrophilicity, and/or the amphipathic nature of the amino acid residues involved. The 20 naturally occurring amino acids can be grouped into the following six standard amino acid groups: (1) hydrophobic - Met, Ala, Val, Leu, Ile; (2) neutral hydrophilic - Cys, Ser, Thr, Asn, Gln; (3) acidic - Asp, Glu; (4) basic - His, Lys, Arg; (5) residues that influence chain orientation - Gly, Pro; and (6) aromatic: Trp, Tyr, Phe. Accordingly, as used herein, the term "conservative substitutions" means an exchange of an amino acid by another amino acid listed within the same group of the six standard amino acid groups shown above. For example, the exchange of Asp by Glu retains one negative charge in the so modified polypeptide. In addition, glycine and proline may be substituted for one another based on their ability to disrupt alpha-helices. Some preferred conservative substitutions within the above six groups are exchanges within the following sub-groups: (i) Ala, Val, Leu and Ile; (ii) Ser and Thr; (iii) Asn and Gln: (iv) Lys and Arg; and (v) Tyr and Phe. Given the known genetic code, and recombinant and synthetic DNA techniques, the skilled scientist readily can construct DNAs encoding the conservative amino acid variants. Synonymous or silent mutations, although not altering the amino acid sequence of the encoded protein directly, can still influence splicing accuracy or efficiency.

As used herein, "non-conservative substitutions" or "non-conservative amino acid exchanges" are defined as exchanges of an amino acid by another amino acid listed in a different group of the six standard amino acid groups (1) to (6) as shown above. Typically, the GmSHC derivatives of the present disclosure are prepared using non-conservative substitutions that alter the biological function of the wild-type GmSHC.

In various embodiments, the amino acid alteration or combination of amino acid alterations enhances the activity of the GmSHC derivative for converting homofarnesol to ambrox compared to wild-type GmSHC, which does not have the amino acid alteration or combination of amino acid alterations. Protein modeling may be used to guide such substitutions, deletions, or insertions in the GmSHC reference sequence. For example, a structural model of the GmSHC amino acid sequence may be created using the coordinates for the A. *acidocaldarius* SHC. Such a homology model is useful for directing improvement of GmSHC enzyme for converting homofarnesol to ambrox, such as a higher production of ambrox upon contact with a homofarnesol substrate than the reference wild-type enzyme.

Amino acid alterations such as amino acid substitutions may be introduced using known protocols of recombinant gene technology including PCR, gene cloning, site-directed mutagenesis of cDNA, transformation of host cells, and *in vitro* transcription, which may be used to introduce such changes to the GmSHC sequence resulting in a GmSHC derivative enzyme. The derivatives can then be screened for GmSHC functional activity.

The GmSHC derivative may have from about 1 to about 45 amino acid alterations, about 1 to about 40 amino acid alterations, about 1 to about 35 amino acid alterations, about 1 to about 30 amino acid alterations, about 1 to about 25 amino acid alterations, from about 1 to about 20 amino acid alterations, about 1 to about 15 amino acid alterations, about 1 to about 10 amino acid alterations, or from about 1 to about 5 amino acid alterations relative to the amino acid sequence of the reference (or wild-type) GmSHC sequence according to SEQ ID NO:2.

Alternatively, the GmSHC derivative can have at least 5, at least 10 amino acid, or at least 15 amino acid alterations relative to the amino acid sequence of the reference (or wild-type) GmSHC sequence according to SEQ ID NO:2, but ideally not more than about 30 or 40 amino acid alterations. In various embodiments, the GmSHC derivative may have about 1 amino acid alteration, about 2 amino acid alterations, about 3 amino acid alterations, about 4 amino acid alterations, about 5 amino acid alterations, about 6 amino acid alterations, about 7 amino acid alterations, about 8 amino acid alterations, about 9 amino acid alterations, about 10 amino acid alterations, about 11 amino acid alterations, about 12 amino acid alterations, about 15 amino acid alterations, about 20 amino acid alterations, about 25 amino acid alterations, about 30 amino acid alterations, about 35 amino acid alterations, about 40 amino acid alterations, about 45 amino acid alterations, or about 50 amino acid alterations relative to the reference GmSHC.

In these or other aspects, the GmSHC derivative shares at least about 50% sequence identity, at least about 55% sequence identity, at least about 60% sequence identity, at least about 65% sequence identity, at least about 70% sequence identity, at least about 75% sequence identity, at least about 80% sequence identity, at least about 85% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, or at least 99% sequence identity to reference GmSHC (SEQ ID NO:2).

In some aspects, a GmSHC derivative includes amino acid alterations at one or more of positions 166, 222, 223, 226, 227, 242, 249, 504, 574, 640, 641, 676, 677 or 682 relative to SEQ ID NO:2. In some embodiments, a GmSHC derivative has one or more of the following amino acid alterations: P166X (silent), V222X (substitution), K223X (substitution), E226X (substitution), D227X (substitution), S242X (substitution), R249X (silent), R504X (substitution), A574X (silent), L640X (substitution), P641X (substitution), M676X (substitution), M677X (substitution) and/or S682X (substitution) relative to SEQ ID NO:2, wherein:
X in P166X is P;
X in V222X is A, C, D, E, F, G, H, I, K, L, M, N, P, Q, R, S, T, W or Y;
X in K223X is A, C, D, E, F, G, H, I, L, M, N, P, Q, R, S, T, V, W or Y;
X in E226X is A, C, D, F, G, H, I, K, L, M, N, P, Q, R, S, T, V, W or Y;
X in D227X is A, C, E, F, G, H, I, K, L, M, N, P, Q, R, S, T, V, W or Y;
X in S242X is A, C, D, E, F, G, H, I, K, L, M, N, P, Q, R, T, V, W or Y;
X in R249X is R;
X in R504X is A, C, D, E, F, G, H, I, K, L, M, N, P, Q, S, T, V, W or Y;
X in A574X is A;
X in L640X is A, C, D, E, F, G, H, I, K, M, N, P, Q, R, S, T, V, W or Y;
X in P641X is A, C, D, E, F, G, H, I, K, L, M, N, Q, R, S, T, V, W or Y;
X in M676X is A, C, D, E, F, G, H, I, K, L, N, P, Q, R, S, T, V, W or Y;
X in M677X is A, C, D, E, F, G, H, I, K, L, N, P, Q, R, S, T, V, W or Y; and
X is S682X is A, C, D, E, F, G, H, I, K, L, M, N, P, Q, R, T, V, W or Y.

Ideally, a GmSHC derivative has one or more of the following amino acid alterations: P166X (silent), V222X (substitution), K223X (substitution), E226X (substitution), D227X (substitution), S242X (substitution), R249X (silent), R504X (substitution), A574X (silent), L640X (substitution), P641X (substitution), M676X (substitution), M677X (substitution) and/or S682X (substitution) relative to SEQ ID NO:2, wherein
X in P166Z is P;
X in V222X is D, E, N, Q, H, K, or R;
X in K223X is S, C, U, T, or M;
X in E226X is G, A, V, L, or I;
X in D227X is S, C, U, T, or M;
X in S242X is H, K, or R;
X in R249X is R;
X in R504X is S, C, U, T, or M;
X in A574X is A;
X in L640X is G, A, V, L, or I;
X in P641X is S, C, U, T, or M;
X in M676X is G, A, V, L, or I;
X in M677X is D, E, N, or Q; and
X is S682X is H, K, or R.

Most preferably, the GmSHC derivative has one or a combination of the following amino acid substitutions: P166P (silent), V222Q or V222R, K223S, E226V, D227T, S242R, R249R (silent), R504C, A574A (silent), L640G, P641S, M676L, M677E and/or S682R relative to SEQ ID NO:2.

In some aspects, the GmSHC derivative has one or a combination of the following amino acid alterations: P166X (silent), V222X (substitution), K223X (substitution), E226X (substitution), D227X (substitution), S242X (substitution), R249X (silent), R504X (substitution), A574X (silent), L640X (substitution), P641X (substitution), M676X (substitution), M677X (substitution) and/or S682X (substitution), where X is defined as above, in combination with one or more of the following amino acid substitutions: V45I, V45Q, V45L, E46H, E46Q, Q54E, S86A, F139L, Y142R, Q178E, M184A, M184L, M184I, M184V, R194Q, G239V, I278V, T326S, L335F, E386Q, I455T, F460A, Q603H, G623A, G623V, F624Y, F624A, L656E and/or Y658F relative to SEQ ID NO:2.

In other aspects, the GmSHC derivative has one or a combination of the following amino acid substitutions: 166, 222, 223, 226, 227, 242, 249, 504, 574, 640, 641, 676, 677 and/or 682 in combination with one or more of the following amino acid substitutions: 45, 46, 54, 86, 139, 142, 178, 184, 194, 239, 278, 326, 335, 386, 455, 460, 603, 623, 624, 656 and/or 658 relative to SEQ ID NO:2.

In a further aspect, the GmSHC derivative has one or a combination of the following amino acid substitutions: P166P (silent), V222Q or V222R, K223S, E226V, D227T, S242R, R249R (silent), R504C, A574A (silent), L640G, P641S, M676L, M677E and/or S682R in combination with one or more of the following amino acid substitutions: 45, 46, 54, 86, 139, 142, 178, 184, 194, 239, 278, 326, 335, 386, 455, 460, 603, 623, 624, 656 and/or 658 relative to SEQ ID NO:2.

In yet a further aspect, the GmSHC derivative has one or a combination of the following amino acid substitutions: P166P (silent), V222Q or V222R, K223S, E226V, D227T, S242R, R249R (silent), R504C, A574A (silent), L640G, P641S, M676L, M677E and/or S682R in combination with one or more of the following amino acid substitutions: V45I, V45Q, V45L, E46H, E46Q, Q54E, S86A, F139L, Y142R, Q178E, M184A, M184L, M184I, M184V, R194Q, G239V, I278V, T326S, L335F, E386Q, I455T, F460A, Q603H, G623A, G623V, F624Y, F624A, L656E and/or Y658F relative to SEQ ID NO:2.

In certain aspects, the GmSHC derivative has a combination of alterations relative to SEQ ID NO:2 as set out in Table 2, or any combination thereof.

**TABLE 2**

| Combination | Mutations | No. of Mutations |
|---|---|---|
| A | V45I/Q54E/V222Q/T326S/F624Y | 5 |
| B | V45I/Q54E/P166P/T326S/F624Y | 5 |
| C | V45I/Q54E/K223S/D227T/T326S/F624Y | 6 |
| D | V45L/Q54E/M184I/V222Q/D227T/ I278T/T326S/A574A/M676L | 9 |
| E | V45I/Q54E/K223S/T326S/A574A/F624Y | 6 |
| F | V45I/Q54E/T326S/A574A/F624Y | 5 |
| G | V45I/Q54E/D227T/T326S/F624Y/M677E | 6 |
| H | V45I/Q54E/V222Q/T326S/F624Y/M676L | 6 |
| I | V45I/Q54E/V222R/T326S/F624Y | 5 |
| J | V45I/Q54E/V222Q/T326S/F624Y/S682R | 6 |
| K | V45I/Q54E/V222Q/R249R/T326S/F624Y | 6 |
| L | V45L/Q54E/M184I/V222Q/D227T/ R249R/I278T/T326S/M676L | 9 |
| M | V45L/Q54E/M184I/V222Q/S242R/ D227T/I278T/T326S/M676L | 9 |
| N | V45I/Q54E/K223S/D227T/T326S/F624Y/ L640G | 7 |
| O | E46Q/Q54E/R194Q/V222Q/D227T/ A574A/F624Y/L640G | 8 |
| P | V45L/Q54E/M184I/V222Q/D227T/ I278T/T326S/M676L/P641S | 9 |
| Q | V45I/Q54E/V222Q/K223S/A574A/F624Y /M676L | 7 |
| R | V45L/Q54E/M184I/V222Q/D227T/ I278T/T326S/A574A/M676L/M677E | 10 |
| S | V45I/Q54E/V222Q/T326S/F624Y/P641S | 6 |
| T | V45L/Q54E/M184I/V222Q/D227T/ I278T/T326S/A574A/P641S/M676L | 10 |
| U | V45I/Q54E/V222R/T326S/F624Y/S682R | 6 |
| V | V45L/Q54E/M184I/V222Q/D227T/ I278T/T326S/R504C/A574A/P641S/M676L | 11 |
| W | V45L/Q54E/M184I/V222Q/D227T/ R249R/I278T/T326S/P641S/M676L | 10 |
| X | V45I/Q54E/M184I/V222Q/D227T/ R249R/I278T/T326S/R504C/M676L | 10 |
| Y | V45L/Q54E/M184I/V222Q/D227T/ I278T/T326S/A574A/P641S/M676L/S682R | 11 |
| Z | V45L/Q54E/M184I/V222R/S242R/ D227T/I278T/T326S/M676L | 9 |
| AA | V45L/Q54E/M184I/V222R/D227T/ I278T/T326S/A574A/P641S/M676L | 10 |

Amino acid substitutions in *A*. *acidocaldarius* SHC (AacSHC) at amino acid positions corresponding to F139L, Y142, I455, G239 and F624 of GmSHC have been shown to increase the activity of the AacSHC enzyme in terms of EEH conversion to ambrox. See WO 2016/170099. Further, F601 of AacSHC has been identified as a highly conserved amino acid residue among the prokaryotic and eukaryotic SHC species. It has been reported that AacSHC derivative F601Y shows a greatly increased Vmax for an oxidosqualene substrate (not squalene); however, F601Y shows a decrease in affinity (*i.e.,* a higher K_{M}) and a decrease in catalytic efficiency/activity (Kcat/K_{M}) relative to the wild-type AacSHC when squalene is used. See Hoshino & Sato (2002) Chem. Commun. (4) :291-301. Notably, the SHC derivative equivalent to F601Y in GmSHC is F624Y. Accordingly, in certain embodiments of this invention, the GmSHC derivative further includes one or more of the above-referenced mutations.

In particular embodiments, the GmSHC derivative has a combination of mutations listed in Table 2. In some embodiments, the GmSHC derivative is a modified GmSHC polypeptide having an amino acid sequence that has up to 4 mutations compared to the wild-type/reference amino acid sequence according to SEQ ID NO:2 and includes at least the substitution Q54E, F624Y, V222R or V222Q relative to SEQ ID NO:2.

Assays for determining and quantifying SHC activity are described herein and are known in the art. By way of illustration, GmSHC and/or GmSHC derivative activity can be determined by incubating purified GmSHC enzyme or extracts from host cells or a complete recombinant host organism that has produced the GmSHC enzyme with an appropriate substrate under appropriate conditions and carrying out an analysis of the reaction products *(e.g.,* by gas chromatography (GC) or HPLC analysis). Further details on GmSHC enzyme activity assays and analysis of the reaction products are provided in the Examples. These assays include producing the GmSHC in recombinant host cells (*e.g., E. coli*).

As used herein, the term "activity" means the ability of an enzyme to react with a substrate to provide a target product. The activity can be determined in what is known as an activity test via the increase of the target product, the decrease of the substrate (or starting materials) or via a combination of these parameters as a function of time. The GmSHC of the present disclosure is characterized by its ability to bioconvert homofarnesol into ambrox.

In embodiments directed to the use of a GmSHC derivative, preferably the GmSHC derivative exhibits a better target yield than the reference GmSHC protein. The term "target yield" refers to the gram of recoverable product per gram of feedstock (which can be calculated as a percent molar conversion rate). In addition, a GmSHC derivative can exhibit a modified (*e.g.,* increased) target productivity relative to the reference GmSHC protein. The term "target productivity" refers to the amount of recoverable target product in grams per liter of fermentation capacity per hour of bioconversion time (*i.e.,* time after the substrate was added). Moreover, a GmSHC derivative can exhibit a modified target yield factor compared to the reference GmSHC protein. The term "target yield factor" refers to the ratio between the product concentration obtained and the concentration of the GmSHC derivative (for example, purified GmSHC enzyme or an extract from the recombinant host cells expressing the GmSHC enzyme) in the reaction medium. In certain embodiments, a GmSHC derivative exhibits at least a 2-, 3-, 4-, 6-, 8-, 10-, 12-, 14-, 16-, 18-, 20-, 25-, 30-, 35-, 40-, 45-, 50-, 55-, 60-, 65-, 70-, 75-, 80-, 85-, 90-, 95-, or 100-fold increase in enzymatic activity (*e.g.,* conversion of homofarnesol to ambrox) relative to the reference GmSHC protein (*e.g.,* SEQ ID NO:2).

A functional homolog of the GmSHC proteins disclosed herein is also included within the scope of this invention. A "functional homolog" is a polypeptide that has sequence similarity to a reference polypeptide, and that carries out one or more of the biochemical or physiological function(s) of the reference polypeptide. A functional homolog and the reference polypeptide may be natural occurring polypeptides, and the sequence similarity may be due to convergent or divergent evolutionary events. As such, functional homologs are sometimes designated in the literature as homologs, or orthologs, or paralogs. Variants of a naturally occurring functional homolog, such as polypeptides encoded by mutants of a wild-type coding sequence, may themselves be functional homologs. Functional homologs can also be created via site-directed mutagenesis of the coding sequence for a polypeptide, or by combining domains from the coding sequences for different naturally-occurring polypeptides ("domain swapping"). Techniques for obtaining functional homologs of the GmSHC enzyme described herein are known and include, inter alia, directed evolution techniques, site-directed mutagenesis techniques and random mutagenesis techniques, which can be used to increase specific activity of the GmSHC enzyme, alter substrate specificity, alter expression levels, or alter subcellular location in a desired manner.

Desirably the GmSHC enzyme and functional homolog share at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% amino acid sequence identity. Preferably, the functional homolog and the reference polypeptide exhibit the indicated sequence identity over a continuous stretch of 20, 30, 40, 45, 50, 60, 70, 80, 90, 100 or more amino acid residues.

To facilitate GmSHC expression and ambrox production and isolation, the GmSHC or GmSHC derivative is expressed in a recombinant host cell. The term "recombinant host," also referred to as a "genetically modified host cell" or "transgenic cell" denotes a host cell that includes a heterologous nucleic acid or the genome of which has been augmented by at least one incorporated DNA sequence. A host cell of the present disclosure may be genetically engineered with a nucleic acid molecule or vector containing a nucleic acid molecule encoding a GmSHC or GmSHC derivative.

The term "nucleic acid molecule," as used herein, refers to polynucleotides of the disclosure which can be DNA, cDNA, genomic DNA, synthetic DNA, or RNA, and can be double-stranded or single-stranded, the sense and/or an antisense strand. The term "nucleic acid molecule" shall particularly apply to the polynucleotide(s) as used herein *(e.g.,* as full-length nucleotide sequence or fragments or parts thereof), which encodes a GmSHC or GmSHC derivative, e.g., SEQ ID NO:1. The term also includes a cDNA; a genomic fragment that lacks at least one of the flanking genes; a fragment of cDNA or genomic DNA produced by polymerase chain reaction (PCR) and that lacks at least one of the flanking genes; a restriction fragment that lacks at least one of the flanking genes; and a DNA encoding a non-naturally occurring protein such as a fusion protein. Fusion proteins can add one or more amino acids to a protein (e.g., a His-tag), usually at the N-terminus of the protein but also at the C-terminus or fused within regions of the protein. Such fusion proteins or fusion vectors encoding such proteins typically provide (i) an increase in the production of recombinant proteins; (ii) an increase in the solubility of the recombinant protein; and/or (iii) an aid in the purification of the recombinant protein by providing a ligand for affinity purification. In certain embodiments, the GmSHC or GmSHC derivative includes a leader sequence to support the expression and/or activity of the GmSHC or GmSHC derivative in a recombinant host cell, *e.g., E. coli.*

The term "nucleic acid molecule" also includes codon optimized sequences suitable for expression in a particular recombinant host cell (*e.g., E. coli* host cell). The term "codon optimized" means a protein coding sequence which has been adapted for expression in a prokaryotic or a eukaryotic host cell, particularly bacterial host cells such as *E. coli* host cells by substitution of one or more or preferably a significant number of codons with codons that are more frequently used in bacterial host cell genes. In this regard, the nucleotide sequence encoding the reference sequence SEQ ID NO:1 and all variants/derivatives thereof may be the original one as found in the source (*e.g.,* GmSHC) or the nucleotide sequence can be codon-optimized for the selected host organisms, such as *e.g., E. coli.*

The term "isolated DNA," as used herein, refers to nucleic acids or polynucleotides isolated from a natural source (*e.g., Gluconobacter morbifer)* or nucleic acids or polynucleotides produced by recombinant DNA techniques, *e.g.,* a DNA construct include a polynucleotide heterologous to a host cell, which is optionally incorporated into the host cell. A chimeric nucleotide sequence may specifically be produced as a recombinant molecule. The term "recombinant," with respect to enzymes, refers to enzymes produced by recombinant DNA techniques, *i.e.,* produced from cells transformed by an exogenous DNA construct encoding the desired enzyme. The term "recombinant" shall specifically apply to assembly of polynucleotides, joining together such polynucleotides or parts thereof, with or without recombination to achieve a cross-over or a gene mosaic. For example, it is performed to join together nucleic acid segments of desired functions to generate a desired combination of functions. A recombinant nucleic acid molecule encoding a polypeptide described herein includes the coding sequence for that polypeptide operably linked, in sense orientation, to one or more regulatory regions suitable for expressing the polypeptide. Because many microorganisms are capable of expressing multiple gene products from a polycistronic mRNA, multiple polypeptides can be expressed under the control of a single regulatory region for those microorganisms, if desired.

A coding sequence and a regulatory region are considered to be operably linked when the regulatory region and coding sequence are positioned so that the regulatory region is effective for regulating transcription or translation of the sequence. Transcriptional/translational regulatory elements include, but are not limited to, inducible and non-inducible, constitutive, cell cycle regulated, metabolically regulated promoters, enhancers, operators, silencers, repressors and other elements that are known to those skilled in the art and that drive or otherwise regulate gene expression. Such regulatory elements include, but are not limited to, regulatory elements such as the CUP-1 promoter; the tet-repressor as employed, for example, in the tet-on or tet-off systems; the lac system, and the trp system regulatory elements. By way of example, Isopropyl β-D-1-thiogalactopyranoside (IPTG) is an effective inducer of gene expression in the concentration range of 100 µM to 1.0 mM. This compound is a molecular mimic of allolactose, a lactose metabolite that triggers transcription of the lac operon, and it is therefore used to induce gene expression when the gene is under the control of the lac operator. Another example of a regulatory element which induces gene expression is lactose.

The nucleic acid molecule(s) of the present disclosure can also form part of a hybrid gene encoding additional polypeptide sequences, for example, a sequence that functions as a marker or reporter. Examples of marker and reporter genes including beta-lactamase, chloramphenicol acetyltransferase (CAT), adenosine deaminase (ADA), aminoglycoside phosphotransferase dihydrofolate reductase (DHFR), hygromycin-B-phosphotransferase (HPH), thymidine kinase (TK), lacZ (encoding beta-galactosidase), and xanthine guanine phosphoribosyltransferase (XGPRT). As with many of the standard procedures associated with the practice of the disclosure, skilled artisans will be aware of additional useful reagents, for example, additional sequences that can serve the function of a marker or reporter.

In some embodiments, the present disclosure provides a recombinant nucleic acid molecule encoding wild-type GmSHC or a GmSHC derivative described above, which may be inserted into a vector for expression and optional purification. Such vectors are referred to herein as "expression vectors." Usually expression vectors suitable for DNA recombination techniques are typically of the plasmid type. An expression vector includes a recombinant nucleic acid molecule encoding wild-type GmSHC or a GmSHC derivative as described herein and the necessary regulatory regions suitable for expressing the polypeptide. Such vectors include nucleic acid molecules that are not naturally present in the host cell, nucleic acid molecules that are not normally transcribed into RNA or translated into a protein ("expressed") and other genes or nucleic acid molecules which one desires to introduce into the host cell. It will be appreciated that typically the genome of a recombinant host cell described herein is augmented through the stable introduction of one or more recombinant nucleic acid molecules. However, autonomous or replicative plasmids or vectors can also be used within the scope of this disclosure. Moreover, the present disclosure can be practiced using a low copy number, *e.g.,* a single copy, or high copy number plasmid or vector. In certain embodiments, the vector of the present disclosure is a plasmid, phagemid, phage, cosmid, artificial bacterial or artificial yeast chromosome, knock-out or knock-in construct, synthetic nucleic acid molecule or cassette produced in the form of a linear polynucleotide, plasmid, megaplasmid, synthetic or artificial chromosome, such as plant, bacterial, mammalian or yeast artificial chromosome.

According to this invention, the GmSHC or a GmSHC derivative encoded by the recombinant nucleic acid molecule is constitutively or inducibly expressed within the cell upon introduction of the vector. Microbial cells are transformed with a vector encoding the GmSHC or a GmSHC derivative using standard transforming techniques. In a suitable embodiment, DNA providing an origin of replication is included in the vector. The origin of replication may be suitably selected by the skilled person. Depending on the nature of the genes, a supplemental origin of replication may not be required if sequences are already present with the genes or genome that are operable as origins of replication themselves.

With the context of the present invention, a microbial cell (e.g., a bacterial or yeast cell) is transformed, when an exogenous or heterologous DNA has been introduced inside the cell. The transforming DNA may or may not be integrated, *i.e.,* covalently linked into the genome of the cell. In prokaryotes, and yeast, for example, the transforming DNA may be maintained on an episomal element such as a plasmid. With respect to eukaryotic cells, a stably transfected cell is one in which the transfected DNA has become integrated into a chromosome so that it is inherited by daughter cells through chromosome replication. This stability is demonstrated by the ability of the eukaryotic cell to establish cell lines or clones including of a population of daughter cells containing the transforming DNA.

Host cells that may be used for the purposes of this disclosure include, but are not limited to, prokaryotic cells such as bacteria (*e.g., E. coli* and *B. subtilis*), which can be transformed with, for example, recombinant bacteriophage DNA, plasmid DNA, bacterial artificial chromosome, or cosmid DNA expression vectors containing the GmSHC nucleic acid molecules of the disclosure; simple eukaryotic cells like yeast (for example, *Saccharomyces* and *Pichia*), which can be transformed with, for example, recombinant yeast expression vectors containing the polynucleotide molecule of the disclosure; insect cells *(e.g.,* a baculovirus insect cell expression system); human cells *(e.g.,* HeLa, CHO and Jurkat), and plant cells (*Arabidopsis* and tobacco). Depending on the host cell and the respective vector used to introduce the nucleic acid molecule of the disclosure, the nucleic acid molecule can integrate, for example, into the chromosome or the mitochondrial DNA or can be maintained extrachromosomally, for example, episomally, or can be only transiently harbored by the cell.

In embodiments pertaining to a eukaryotic cell, preferably the cell is a fungal, mammalian or plant cell. Suitable eukaryotic cells include, for example, without limitation, mammalian cells, yeast cells (*e.g., Saccharomyces, Candida, Kluyveromyces, Schizosaccharomyces, Yarrowia, Pichia* and *Aspergillus*), or insect cells (including Sf9), amphibian cells (including melanophore cells), or worm cells including cells of *Caenorhabditis* (including *Caenorhabditis elegans*). Suitable mammalian cells include, for example, without limitation, COS cells (including Cos-1 and Cos-7), CHO cells, HEK293 cells, HEK293T cells, or other transfectable eukaryotic cell lines.

In embodiments pertaining to prokaryotes, preferably the cell is *E. coli, a Bacillus* sp., or *Streptomyces* sp. Preferably the *E. coli* host cell is an *E. coli* host cell that is recognized by the industry and regulatory authorities as suitable for recombinant protein expression (including but not limited to an *E. coli* K12 host cell or *E. coli* BL21 host cell). In certain embodiments, the recombinant host cell of this invention is *E. coli.* There are libraries of mutants, plasmids, detailed computer models of metabolism and other information available for *E. coli,* allowing for rational design of various modules to enhance product yield. Accordingly, in certain embodiments, a recombinant *E. coli* expressing a nucleic acid molecule encoding a GmSHC or GmSHC derivative coding sequence is provided for converting homofarnesol to ambrox.

Another preferred host cell to use with the present disclosure is *S. cerevisiae,* which is widely used in synthetic biology. Thus, the recombinant host cell may be *S. cerevisiae.* There are libraries of mutants, plasmids, detailed computer models of metabolism and other information available for *S. cerevisiae,* allowing for rational design of various modules to enhance product yield. In addition, methods are known for making recombinant *S. cerevisiae* microorganisms. Accordingly, in certain embodiments, a recombinant *S. cerevisiae* expressing a nucleic acid molecule encoding a GmSHC or GmSHC derivative coding sequence is provided for converting homofarnesol to ambrox.

Culturing of cells is performed in a conventional manner. The culture medium contains a carbon source, at least one nitrogen source and inorganic salts, and vitamins are added to it. The constituents of this medium can be the ones which are conventionally used for culturing the species of host cell in question. Carbon sources of use in the methods described herein include any molecule that can be metabolized by the recombinant host cell to facilitate growth and/or production of ambrox. Examples of suitable carbon sources include, but are not limited to, sucrose *(e.g.,* as found in molasses), fructose, xylose, glycerol, glucose, cellulose, starch, cellobiose or other glucose containing polymer.

In embodiments employing *E. coli,* a defined minimal medium such as M9A may be used for cell cultivation. The components of M9A medium include: 14 g/L KH₂PO₄, 16 g/L K₂HP0₄, l g/L Na₃Citrate-2H₂0, 7.5 g/L (NH₄)₂S0₄, 0.25 g/L MgSO₄·7H₂0, 0.015 g/L CaCl₂·2H₂0, 5 g/L glucose and 1.25 g/L yeast extract. In another embodiment of this disclosure, a nutrient-rich medium such as LB is used. The components of LB medium include: l0 g/L tryptone, 5 g/L yeast extract, 5 g/L NaCl. Other examples of Mineral Medium and M9 Mineral Medium are disclosed, for example, in US 6,524,831 and US 2003/0092143.

In embodiments employing yeast as a host, for example, carbon sources such as sucrose, fructose, xylose, ethanol, glycerol, and glucose are suitable. The carbon source can be provided to the host organism throughout the cultivation period or alternatively, the organism can be grown for a period of time in the presence of another energy source, e.g., protein, and then provided with a source of carbon only during the fed-batch phase.

The suitability of a recombinant host cell for use in the methods of the present disclosure may be determined by simple test procedures using well-known methods. For example, the host cell to be tested may be propagated in a rich medium (e.g., LB-medium, Bacto-tryptone yeast extract medium, nutrient medium and the like) at a pH, temperature and under aeration conditions commonly used for propagation of the microorganism. Once a recombinant host cell is identified as producing the desired products of bioconversion, the products are typically produced by a production host cell line on the large scale by suitable expression systems and fermentations, *e.g.,* by microbial production in cell culture.

The recombinant host cell may be grown in a batch, fed batch or continuous process or combinations thereof. As used herein, the term "batch cultivation" is a cultivation method in which culture medium is neither added nor withdrawn during the cultivation. By comparison, the term "fed-batch" means a cultivation method in which culture medium is added during the cultivation but no culture medium is withdrawn. Typically, the recombinant host cell is grown in a culture system, wherein the recombinant host cells are grown in fermenter at a defined temperature(s) in the presence of a suitable nutrient source, e.g., a carbon source, for a desired period of time to produce sufficient enzyme to bioconvert homofarnesol to ambrox and to produce a desired amount of ambrox including (-)- ambrox. The recombinant host cells may be cultivated in any suitable manner, for example by batch cultivation or fed-batch cultivation. Often, however, higher cumulative production titers can be achieved by implementing a continuous process, such as product removal, substrate feed, and biomass addition or (partial) replacement.

One embodiment of the present disclosure provides a method of producing ambrox in recombinant host cells by providing recombinant host cells expressing wild-type GmSHC or a GmSHC derivative in a culture system, providing homofarnesol to the culture system (e.g., by feeding), converting homofarnesol to ambrox using the GmSHC or GmSHC derivative produced by the recombinant host cells, collecting ambrox and optionally isolating the ambrox (in particular, (-) -ambrox) . In some embodiments, the recombinant host cell also expresses other nucleic acid molecules that serve to enhance the expression of GmSHC or bioconversion pathway for making ambrox.

Another embodiment of the present disclosure provides a method of producing ambrox in recombinant host cells by providing recombinant host cells expressing wild-type GmSHC or a GmSHC derivative in a culture system, providing homofarnesol to the culture system, feeding homofarnesol (e.g., EEH) to the culture system to promote the conversion of homofarnesol to ambrox, collecting ambrox and optionally isolating the ambrox (in particular, (-)-ambrox). In some embodiments, conversion may be enhanced by the adding a solubilizing agent, in particular a non-ionic surfactant or detergent such as Polysorbate 80, Triton X-100 and the like, to the reaction mixture.

The recombinant host cells may be cultured in a number of ways in order to provide cells in suitable amounts expressing the wild-type GmSHC or GmSHC derivative for the subsequent bioconversion step. Since the host cells applicable for the bioconversion step vary broadly (e.g., fungal, bacterial, insect, mammalian and plant cells), culturing conditions are, of course, adjusted to the specific requirements of each species and these conditions are well-known and documented. Any of the art known methods for growing recombinant host cells may be used to produce the cells used in the subsequent bioconversion step of the present disclosure. Typically, the cells are grown to a particular density (measurable as optical density (OD)) to produce a sufficient biomass for the bioconversion reaction.

The cultivation conditions chosen can influence the amount of cells obtained (the biomass) as well as how the biomass becomes a biocatalyst (*i.e.,* a cell or cell fraction containing a wild-type GmSHC or GmSHC derivative). In some embodiments, the biocatalyst is a recombinant whole cell that expresses wild-type GmSHC or GmSHC derivative. In other embodiments, the biocatalyst is a recombinant whole cell suspension or immobilized cell that expresses wild-type GmSHC or GmSHC derivative. In other embodiments, the biocatalyst is a membrane fraction or a liquid fraction prepared from the recombinant host cell that expresses a wild-type GmSHC or GmSHC derivative. The recombinant whole cell producing a wild-type GmSHC or GmSHC derivative include whole cells collected from the fermenter (for the bioconversion reaction) or the cells in the fermenter (which are then used in a one-pot reaction). The recombinant whole cell producing a wild-type GmSHC or GmSHC derivative can include intact recombinant whole cell and/or cell debris. Either way, the wild-type GmSHC or GmSHC derivative is associated with a membrane (such as a cell membrane) in some way in order to receive and/or interact with a substrate (e.g., homofarnesol), which membrane (such as a cell membrane) can be part of or include a whole cell (e.g., a recombinant whole cell). The wild-type GmSHC or GmSHC derivative may also be in an immobilized form (e.g., associated with an enzyme carrier) which allows the wild-type GmSHC or GmSHC derivative to interact with a substrate *(e.g.,* homofarnesol). The wild-type GmSHC or GmSHC derivative may also be used in a soluble form.

In one embodiment, the biocatalyst is produced in sufficient amounts (to create a sufficient biomass), harvested and washed (and optionally stored (e.g., frozen or lyophilized)) before the bioconversion step. In a further embodiment, the cells are produced in sufficient amounts (to create a sufficient biocatalyst) and the reaction conditions are then adjusted without the need to harvest and wash the biocatalyst for the bioconversion reaction. This one step (or "one pot") method is advantageous as it simplifies the process while reducing costs. The culture medium used to grow the cells is also suitable for use in the bioconversion reaction provided that the reaction conditions are adjusted to facilitate the bioconversion reaction.

The optimum pH for growing the cells is in the range of 6.0-7.0. The optimum pH for the bioconversion reaction may be dependent on the SHC enzyme used in the bioconversion reaction, e.g., wild-type GmSHC or GmSHC derivative. The pH is regulated using techniques which are well-known to the skilled person.

While the terms "mixture" or "reaction mixture" may be used interchangeably with the term "medium" in the present disclosure (especially as it relates to a "one pot" reaction), it should be noted that growing the cells to create a sufficient biomass requires a cell culture/fermentation medium but a medium is not required for the bioconversion step as a reaction buffer will suffice at a suitable pH.

The bioconversion methods of the present disclosure are carried out under conditions of time, temperature, pH and solubilizing agent to provide for conversion of the homofarnesol feedstock to ambrox. The pH of the reaction mixture may be in the range of 4 to 8, preferably, 5 to 6.5, more preferably 4.8 to 6.0 for the GmSHC derivative enzymes and in the range of from about pH 5.0 to about pH 7.0 for the wild-type GmSHC enzyme and can be maintained by the addition of buffers to the reaction mixture. The buffer used may be a citrate, phosphate, TRIS (tris(hydroxymethyl)aminomethane), or MES (2-(N-morpholino)ethanesulfonic acid) buffer. In certain embodiments, the buffer is Tris-Cl buffer. The preferred temperature is between from about 15°C and about 45°C, preferably about 20°C and about 40°C The temperature can be kept constant or can be altered during the bioconversion process.

The use of a solubilizing agent, e.g., a surfactant, detergent, solubility enhancer, water miscible organic solvent and the like, may optionally be used to improve in the bioconversion reaction. As used herein, the term "surfactant" means a component that lowers the surface tension (or interfacial tension) between two liquids or between a liquid and a solid. Surfactants may act as detergents, wetting agents, emulsifiers, foaming agents, and dispersants. In certain embodiments, the surfactant is a nonionic surfactant, anionic surfactant, cationic surfactant or amphoteric or zwitterionic surfactant. Examples of nonionic surfactants include, but are not limited to, Triton X-100 (4-(1,1,3,3-Tetramethylbutyl)phenyl-polyethylene glycol, *t-*Octylphenoxypolyethoxyethanol, Polyethylene glycol *tert-*octylphenyl ether), Polysorbate 80, and Polysorbate 20. Exemplary anionic surfactants include, but are not limited to, taurodeoxycholate, sodium taurodeoxycholate, sodium dodecyl sulfate (SDS), and sodium lauryl sulfate (SLS).

According to the methods of this invention, ambrox is produced using a biocatalyst to which the homofarnesol substrate is added. It is possible to add the substrate by feeding using known means (*e.g.,* peristaltic pump, infusion syringe and the like). Homofarnesol is an oil soluble compound and is provided in an oil format. Given that the biocatalyst (microbial cells such as intact recombinant whole cell and/or cell debris and/or immobilized enzyme) is present in an aqueous phase, the bioconversion reaction may be regarded as a three phase system (including an aqueous phase, a solid phase and an oil phase) when homofarnesol is added to the bioconversion reaction mixture. This may also be the case even when solubilizing agent is present.

While some embodiments include the use of whole intact cells or cell extracts, other embodiments include the use of free, optionally purified or partially purified GmSHC enzyme or immobilized GmSHC enzyme for bioconversion of homofarnesol to ambrox. In this respect, when a soluble wild-type GmSHC or a GmSHC derivative is used as a biocatalyst, this is considered a two-phase system.

The number of homofarnesol isomers present may influence the speed of the reaction. It has been demonstrated that a SHC derivative enzyme is capable of biocoverting E,E-homofarnesol to (-)-ambrox from a complex mixture of homofarnesol isomers (e.g., EE:EZ:ZE:ZZ) (see WO 2016/170099). However, a lower conversion rate is typically observed using the complex mixture of homofarnesol isomers, which is consistent with the view that homofarnesol isomers other than EEH may compete with EEH for access to the SHC derivative enzyme and thus may act as competitive inhibitors for the conversion of EEH to (-)-ambrox and/or also act as alternative substrates. Accordingly, the present method is preferably carried out in the presence of a homofarnesol substrate composed of a stereoisomeric mixture of 2-4 isomers, preferably two isomers. In some embodiments, only two isomers of homofarnesol are added to the reaction mixture. In certain embodiments, the homofarnesol substrate is composed of an EE:EZ stereoisomeric mixture. In other embodiments, stereoisomerically pure E,E-homofarnesol is added to the reaction mixture.

The ambrox produced by the method of this invention may be collected, e.g., steam extraction/distillation or organic solvent extraction using a non-water miscible solvent (to separate the reaction products and unreacted substrate from the biocatalyst which stays in the aqueous phase) followed by subsequent evaporation of the solvent to obtain a crude reaction product as determined by gas chromatographic (GC) analysis. Steam extraction/distillation and organic solvent extraction methods are known to those skilled in the art. By way of illustration, the resulting ambrox may be extracted from the whole reaction mixture using an organic solvent such as a non-water miscible solvent *(e.g.,* toluene). Alternatively, the resulting ambrox may be extracted from the solid phase of the reaction mixture (obtained by, *e.g.,* centrifugation or filtration) using a water miscible solvent *(e.g.,* ethanol) or a non-water miscible solvent *(e.g.,* toluene). By way of further example, ambrox is present in the solid phase as crystals or in amorphous form and can be separated from the remaining solid phase (cell material or debris thereof) and the liquid phase also by means of filtration. By way of further example, at a temperature above the melting point of ambrox (approximately 75°C), the ambrox may form an oil layer on top of aqueous phase, wherein the oil layer can be removed and collected. In order to ensure a complete recovery of ambrox after the oil layer is removed, an organic solvent may be added to the aqueous phase containing the biomass in order to extract any residual ambrox contained in, or on or about the biomass. The organic layer can be combined with the oil layer, before the whole is further processed to isolate and purify ambrox. The ambrox may be further selectively crystallized to remove by-products (*i.e.,* isomers other than (-)-ambrox) and any unreacted homofarnesol substrate from the final product. The term "selective crystallization" refers to a process step whereby (-)-ambrox is caused to crystallize from a solvent while the remaining isomers remain dissolved in the crystallizing solvent. In some embodiments, the isolated crystalline material contains only (-)-ambrox product. In other embodiments, the isolated crystalline material contains the other isomers, wherein said isomers are present only in olfactory acceptable amounts.

Examples of suitable water miscible and non-water miscible organic solvents suitable for use in the extraction and/or selective crystallization of (-)-ambrox include, but are not limited to, aliphatic hydrocarbons, preferably those having 5 to 8 carbon atoms, such as pentane, cyclopentane, hexane, cyclohexane, heptane, octane or cyclooctane; halogenated aliphatic hydrocarbons, preferably those having one or two carbon atoms, such as dichloromethane, chloroform, carbon tetrachloride, dichloroethane or tetrachloroethane; aromatic hydrocarbons, such as benzene, toluene, the xylenes, chlorobenzene or dichlorobenzene; aliphatic acyclic and cyclic ethers or alcohols, preferably those having 4 to 8 carbon atoms, such as ethanol, isopropanol, diethyl ether, methyl tert-butyl ether, ethyl tert-butyl ether, dipropyl ether, diisopropyl ether, dibutyl ether, tetrahydrofuran; or esters such as ethyl acetate or n-butyl acetate or ketones such as methyl isobutyl ketone or dioxane or mixtures of these. The solvents that are especially preferably used are the above-mentioned heptane, methyl tert-butyl ether (also known as MTBE, tertiary butyl methyl ether and iBME), diisopropyl ether, tetrahydrofuran, ethyl acetate and/or mixtures thereof. Preferably, a water miscible solvent such as ethanol is used for the extraction of (-)-ambrox from the solid phase of the reaction mixture. The use of ethanol is advantageous because it is easy to handle, it is nontoxic and it is environmentally friendly.

In certain embodiments, the final product is isolated (-)-ambrox. The term "isolated" as used with reference to (-)-ambrox, refers to a bioconversion product that has been separated or purified from components which accompany it. An entity that is produced in a cellular system different from the source from which it naturally originates is "isolated" because it will necessarily be free of components which naturally accompany it. The degree of isolation or purity can be measured by any appropriate method, *e.g.,* gas chromatography (GC), HPLC or NMR analysis. In some embodiments, the end product ((-)-ambrox) is isolated and purified to homogeneity, *e.g.,* at least 80%, 81%, 82%, 83%, 84%, 85%, 86%, 87%, 88%, 89%, or 89.5% pure or 90%, 91%, 92%, 93%, 94%, 95%, 96%, 97%, 98%, 99%, or 99.5% pure.

The olfactive purity of the final (-)-ambrox product may be determined using a 10% ethanol extract in water or by testing the crystalline material. The final (-)-ambrox product may be tested against a commercially available reference of (-)-ambrox product for its olfactive purity, quality and its sensory profile. The (-)-ambrox material can also be tested in application studies by experts in order to determine if the material meets the specifications with respect to its organoleptic profile.

The activity of the GmSHC enzyme is defined via the reaction rate (amount of product/(amount of product + amount of remaining starting material)) x 100) in mol percent. Preferably, the bioconversion of EEH into (-)-ambrox in the presence of wild-type GmSHC or a GmSHC derivative enzyme, or in the presence of a recombinant host cell that expresses a wild-type GmSHC or a GmSHC derivative, provides an (-)-ambrox yield of 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, given in mol percent and based on the mols of EEH employed; especially preferably, the yield is between 5 and 100, 10 and 100, 20 and 100, 25 and 100, 30 and 100, 35 and 100, in particular between 40 and 100, 45 and 100, 50 and 100, 60 and 100, 70 and 100.

In a preferred embodiment of the invention, the yield and/or the reaction rate are determined over a defined time period of, for example, 4, 6, 8, 10, 12, 16, 20, 24, 36 or 48 hours, during which EEH is converted into (-)-ambrox by a recombinant host cell harboring a nucleic acid molecule encoding a wild-type GmSHC or a GmSHC derivative enzyme according to the present disclosure. In a further embodiment, the reaction is carried out under defined conditions of, for example, 25°C, 30°C, 40°C, 50°C or 60°C.

The bioconversion process for making (-)-ambrox from homofarnesol in a recombinant strain of *E. coli* harboring a nucleic acid molecule encoding a wild-type GmSHC or a GmSHC derivative enzyme can offer a low cost and industrially economical process for (-)-ambrox production.

Desirably, the amount of (-)-ambrox produced is in the range of about 1 mg/L to about 20,000 mg/L (20 g/L) or higher such as from about 20 g/L to about 200 g/L or from 100 to 200 g/L, preferably about 125 g/L or 150 g/L.

Various applications for (-)-ambrox include, but are not limited to, a fine fragrance or a consumer product such as fabric care, toiletries, beauty care and cleaning products including essentially all products where the currently available ambrox ingredients are used commercially, including but not limited to, Ambrox (Henkel), Amberlyn (Quest), and Norambrenolide Ether (Pacific), and those products sold under the trademarks AMBROX^{®} (Firmenich), AMBROFIX^{®} (Givaudan), CETALOX^{®} Laevo (Firmenich), and/or AMBERMOR^{®} (Aromor). The selective crystallization of (-)-ambrox may be influenced by the presence of unreacted homofarnesol substrate and also the ratio of (-)-ambrox to the other detectable isomers. Even if only 10% conversion of the homofarnesol substrate to (-)-ambrox is obtained, the selective crystallization of (-)-ambrox is still possible.

The following non-limiting examples are provided to further illustrate the present invention.

### Example 1: Insertion of a PelB Leader Sequence

As one approach to increasing the enzyme activity of GmSHC, a pelB leader sequence was inserted into the pET28b(+) vector containing nucleic acids encoding GmSHC. An oligonucleotide encoding the pelB leader sequence was prepared with *Nco*I and *NdeI* compatible ends for insertion into the pET28b(+) vector. The pET28b(+) vector was digested with *NcoI* and NdeI and used in an overnight ligation reaction with the pelB leader sequence oligonucleotide. The ligation reaction was purified and used in the transformation of electrocompetent *E. coli.* A random sample of the resulting transformant *E. coli* was assessed by colony PCR with complimentary oligonucleotide primers 'pelB-SHC-Fw' and 'pET-XhoI-Rev' to determine whether the ligation reactions were successful. A clone was identified as containing an insert of the correct size. Subsequent DNA sequence analysis confirmed the insertion of a pelB leader sequence into the pET28b(+) vector.

### Example 2: Expression Analysis of the PelB-GmSHC Fusion Protein

The plasmid containing the pelB-GmSHC clone was used to transform *E. coli* BL21(DE3) for expression of the fusion protein. Following transformation, a single colony clone was isolated and used to inoculate 10 mL of LB medium + kanamycin. The 10 mL culture was incubated at 37°C, with shaking at 200 rpm overnight. The overnight culture was used to inoculate a flask containing 1 L of LB medium + kanamycin, which was incubated at 37°C, with shaking at 200 rpm for 6 hours prior to induction. Induction of protein expression was initiated by the addition of 1 mL of 1 M IPTG. Following induction, the incubator temperature was dropped to 25°C and the culture was left overnight with shaking at 200 rpm. An aliquot (1.5 mL) of the 25°C overnight culture was removed for expression analysis by SDS-PAGE, the remaining culture was harvested by centrifugation for further work. From SDS-PAGE analysis, it was observed that a pelB-GmSHC fusion protein of the correct size was expressed.

### Example 3: Whole Cell Screening Assays

The pelB leader sequence was included to facilitate the transport of the GmSHC enzyme into the *E. coli* periplasmic space thereby making the GmSHC enzyme more available to substrates in the environment surrounding the cells. Accordingly, screening assays were carried to analyze the conversion of homofarnesol to ambrox by whole cell suspensions containing pelB-GmSHC as compared to GmSHC. Reactions included whole cells, 100 µl of 1 M Sodium Citrate, pH 4.9, 100 µl of 100 mM homofarnesol in Solubilization Buffer (0.05 M Tris-Cl, pH 8.0, 0.01 M MgCl₂, 1 % v/v TRITON X-100) and 800 µl of Solubilization Buffer. The reactions were incubated at 37°C, 200 rpm and samples were removed after 16 and 80 hours. The samples were extracted with 2 volumes of n-heptane prior to GC analysis. The average % area conversion per mg of whole cell was calculated. The results indicated that whereas the wild-type GmSHC cell suspensions provided an average 0.033% area per mg whole cell per hour, pelB-GmSHC cell suspensions did not result in any conversion of homofarnesol to ambrox. Accordingly, the pelB leader sequence appeared to adversely affect the activity of GmSHC.

As SHC enzymes are co-factor independent, it was posited that GmSHC may retain activity post-reaction. To determine whether any activity was retained, cells from the 16-hour time point described above were removed and resuspended in 0.5 mL of fresh reaction mixture. These '2^{nd} pass' reactions were then incubated at 37°C, 200 rpm for approximately 64 hours. After 64 hours, the reactions were extracted with 2 volumes of n-heptane for GC analysis. The comparison of the '1^{st} pass' and '2^{nd} pass' indicated minimal degradation of activity following repeat exposure to fresh reaction mixture (0.033% at 1^{st} pass versus 0.037% at 2^{nd} pass). Accordingly, these data indicate that the whole cells can be reused/recycled to perform repeat conversions, which may prove advantageous to minimizing the overall cost of the process.

### Example 4: Conversion of Homofarnesol to Ambrox in the Fermentation

As demonstrated above, whole cells expressing GmSHC bioconverted homofarnesol to ambrox. Accordingly, it was determined whether conversion in the fermentation could be achieved. Cells expressing wild-type GmSHC or *R. palustris* SHC (RpSHC; WO 2010/139719) were grown and expressed in the following manner. Overnight 10 mL (LB medium + kanamycin, 37°C, 200 RPM) starter cultures of the cells harboring nucleic acids encoding GmSHC and RpSHC were used to inoculate 1L of LB medium + kanamycin. The 1 L cultures were incubated at 37°C, 200 rpm for approximately 4 hours. Subsequently, 1 mL of 1M IPTG was added to the cultures to induced SHC protein expression and the incubation temperature of the culture was reduced to 25°C for the overnight incubation.

To confirm expression of GmSHC and RpSHC, analysis by SDS-PAGE was performed on the cell culture. Aliquots (1.5 mL) of the overnight culture were removed and centrifuged at 14,500 rpm for 2 minutes. The supernatant was discarded and the cell pellets were resuspended in 200 µL of SDS loading buffer. The resuspended cells were then heated to 95°C for 5 minutes. Following a brief centrifugation, 14,500 rpm for 10 seconds, samples of the SHCs in loading buffer were placed into the wells of a precast 4-20% SDS-PAGE gel. The results of this analysis indicated that both GmSHC and RpSHC were expressed.

Following confirmation of expression of GmSHC and RpSHC, duplicate reactions were prepared as provided in Table 3.

**TABLE 3**

| Reaction Contents | Set A | Set B |
|---|---|---|
| Cell Culture* | 850 µL | 850 µL |
| 1 M Na Citrate, pH 4.9 | 100 µL | 100 µL |
| 100 mM Homofarnesol in 0.05 M Tris-Cl, pH 8.0, 0.01 M MgCl₂, 1 % v/v TRITON X-100 | 50 µL | - |
| *100 mM Homofarnesol in 0.1M Na Citrate, pH 6.5, 2% Taurodeoxycholate | - | 50 µL |

| | | |
|---|---|---|
| *Emulsion described in WO 2010/139719. | | |

The reactions were prepared and incubated at 37°C, 200 rpm. Samples were removed after 16 hours incubation and extracted with 2 volumes of n-heptane for GC analysis. After 40 hours, the remaining reaction mixture was centrifuged to pellet the cells and the supernatant was extracted with 2 volumes of n-heptane for GC analysis. The averages of the % area conversion per hour are presented in Table 4.

**TABLE 4**

| SHC Culture | Set A | Set B |
|---|---|---|
| GmSHC | 0.46 | 0.00 |
| RpSHC | 0.10 | 0.00 |

The results presented in Table 4 demonstrate conversion of homofarnesol to ambrox by the SHCs in cell culture medium. The results also demonstrate that homofarnesol in TRITON X-100 was more readily converted to ambrox than as an emulsion in taurodeoxycholate.

### Example 5: Generation of GmSHC Derivatives

Three different approaches were taken to generate GmSHC derivatives: rational mutagenesis (site-directed mutagenesis), semi-rational mutagenesis (via site-saturation library), and random mutagenesis (error prone PCR). Mutants were expressed in a heterologous system and screened by GC.

*Homology Modeling.* The three-dimensional structure of GmSHC was build using homology modeling. The templates used were the crystals 1GSZ (Lenhart, et al. (2002) Chem. Biol. 9:639-45) and 3SQC (Wendt, et al. (1999) J. Mol. Biol. 286:175-87), which share 44% and 43% of sequence identity with 95% of GmSHC sequence.

*Molecular Docking.* The ground state representations of homofarnesol was then docked to the active center of the GmSHC structure. This was achieved by defining a 3D grid box centered in the protonated oxygen atom of the first proton donor. This grid box identifies the active center pocket area where the substrates conformations will be sampled during the molecular docking run. Then molecular docking was performed using the Lamarckian genetic algorithm (LGA; Morris, et al. (1998) J. Comput. Chem. 19:1639-62; Morris, et al. (2009) J. Comput. Chem. 30:2785-91). A total of 1000 LGA runs were carried out per system. The population was 300, the GA elitism=1, the maximum number of generations was 27000 and the maximum number of energy evaluations was 2500000. Accordingly, for each LGA run the first generation started with a population of 300 random substrates conformations. The best substrate conformation in the current population automatically survives into the next generation (GA elitism=1). As such, the next generation population starts with the fittest substrate conformation from the previous generation plus another 299 conformations. The LGA run stops when the number of maximum generations or energy evaluations are reached. For each LGA run, one substrate conformation was obtained. Substrate conformations were then sorted according to energy and root mean square deviation. The top ranked structure corresponded to the lowest binding energy structure of the most populated cluster with the lowest mean binding energy.

*SHC Structural Analysis and Catalytic Mechanism.* SHCs are integral monotopic membrane proteins which adopt a dimeric 3D arrangement. Each monomer is characterized by eight QW motifs (Sato, et al. (1998) Biosci. Biotechnol. Biochem. 62:407-11) that tightly connect numerous α-helices building up two highly stable α/α-barrels domains (Wendt, et al. (1999) J. Mol. Biol. 286:175-87). The active center cavity is buried within the two α/α-barrels domains and its access is possible through an inner hydrophobic channel. For AaSHC, the channel and the active center cavity are separated by a narrow constriction constituted by residues F166, V174, F434, and C435, which is responsible for substrate recognition (Lenhart, et al. (2002) Chem. Biol. 9:639-45). For GmSHC, those residues correspond to F176, M184, F457 and C458. Unless indicated otherwise, the position of the amino acid residues provided with respect to GmSHC are with reference to SEQ ID NO:2. At the top of the activity center cavity, the residues that constitute the conserved DXDD motif (Wendt, et al. (1999) J. Mol. Biol. 286:175-87) are observed. One of those residues is D396, the first proton donor, which initiates the cyclization by donating a proton to the double bond 2 and 3 (Scheme 2). In GmSHC, the oxygen atom of D396 is 4.6Ä from the carbon 3 of the double bond 2,3.

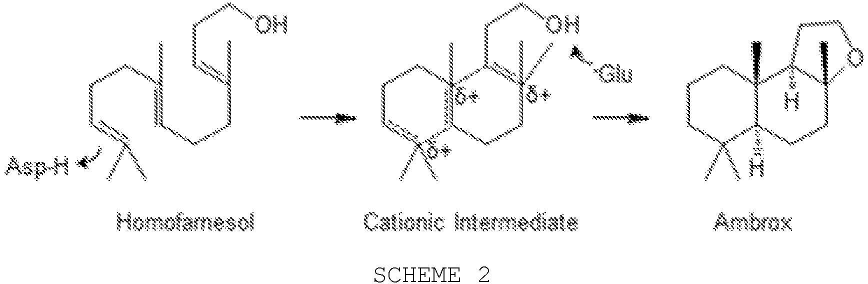

The DXDD motif is followed by tryptophan and phenylalanine residues that are responsible for stabilizing the cationic intermediates by strong cation-π interactions (Dougherty (1996) Science 271:163-168). On the bottom of the cavity is a Glutamate residue, the last proton acceptor, which may receive a proton from the hydroxyl group and lead to the closing of the third ring and formation of the product, ambrox. Structural analysis of GmSHC indicates that this enzyme possesses two possible last proton acceptors. However, according to the docking results, E386 of GmSHC is the most likely the last proton acceptor. The distance between the homofarnesol hydroxyl oxygen and E386 is just 3.5Ä. Clearly this disposition of the last proton acceptor plays an important role in the catalytic efficacy of this enzyme.

Using the molecular model, GmSHC residues that establish the cation-pi interactions responsible for the stabilization of the cationic intermediate and the other main catalytic residues were determined. Notably, most of the important catalytic residues are conserved with other RpSHC enzyme (WO 2010/139719). The main differences include: a) the GmSHC active center is residue 45; b) GmSHC residue 184, together with residues 176, 457 and 458, is responsible for the narrow constriction between the hydrophobic channel and the active center cavity, which is associated with substrate selectivity; and c) the pattern of QW motifs is somewhat different.

*Structural Hotspots.* Based on the molecular modeling and molecular docking results, the following active center structural hotspots, which when mutated can improve the chemical step of the enzyme catalysis, were identified: residues V45, E46, Q54, F176, M184, F457, C458, W179, I278, Q279, T326, F385, E386, D397, F443, F460, F624, F654 and L656.

*Specificity Determining Positions and Conserved Residues.* The specificity determining positions indicate which residues coordinately evolved within a subgroup of proteins of a family that shares a given catalytic specificity. Thus, it allows following the evolutionary process associated with acquiring a diversity of biological functions within the same family of proteins. Specificity determining positions were calculated from a multiple sequence alignment containing 1000 homologous sequences using the algorithms of Xdet.

*Evolution of GmSHC.* To improve the catalytic conversion of homofarnesol to ambrox, GmSHC was modified to (1) improve the Michaelis-Menten complex; (2) introduce mutations that can increase the cation-π stabilization of the carbocation intermediate, based on the structural and coevolution hotspots; (3) open the catalytic cavity by mutating the residues that are only essential for the catalysis of the 5-ring native substrate, squalene; (4) mutate the residues that assist the last proton acceptor in order to facilitate product formation; (5) alter the active center; (6) mutate residues responsible for the narrow constriction between the hydrophobic channel and the active center cavity; (7) and increase the QW motifs.

GmSHC variants designed to improve the Michaelis-Menten complex, and increase the cation-π stabilization of the carbocation intermediate, open the catalytic cavity by mutating the residues that are only essential for the catalysis of the 5-ring native substrate, squalene, mutate the residues that assist the last proton acceptor in order to facilitate product formation and alter the active center were tested *in silico* using molecular docking. The results of this analysis are presented in Table 5.

**TABLE 5**

| SHC mutant | Calculated Affinity | Comments |
|---|---|---|
| WT | -6.01 | -- |
| I278F | -6.30 | Substrate is closer to the first proton donor. |
| V45I | -6.05 | Similar to WT. |
| V45F | -6.38 | Substrate is closer to the first proton donor. |
| L656W | -6.14 | Substrate is closer to the first proton donor; Different last proton acceptor. |
| L656F | -6.22 | Substrate is closer to the first proton donor; Different last proton acceptor. |
| W179A | -6.45 | Substrate is closer to the first proton donor; Different last proton acceptor. |
| I278A | -6.26 | Substrate is closer to the first proton donor. |
| F654Y | | Designed to increase cation-n interactions. Substrate pose similar to WT. |
| I278N | -6.43 | Substrate is closer to the first proton donor; Same last proton acceptor but the substrate hydroxyl also interacts with I278N. |
| T326N | -6.15 | Substrate is closer to the first proton donor; Same last proton acceptor but now T326N improves polarity surrounding the last proton acceptor. |
| L656E | -6.06 | Substrate is closer to the first proton donor; L656E last proton acceptor. |
| G623A | -6.41 | Substrate is closer to the first proton donor and to the last proton acceptor. |
| F460A | -6.13 | Opens the lower part of the active center cavity. The substrate pose is similar to WT. |
| F443Y | -5.94 | Designed to increase cation-n interactions. |
| E386H | -6.11 | Different last proton acceptor. |

Additional mutations addressing each of the modifications indicated above are listed Table 6.

**TABLE 6**

| Functional Change | GmSHC Mutation |
|---|---|
| (1) improve the Michaelis-Menten complex; and | V45I |
| | V45L |
| (5) alter the active center | V45Q |
| | V45A |
| | E46Q |
| | E46A |
| | E46H |
| | Q54E |
| | Q54H |
| | G623A |
| | G623V |
| | F385Y |
| | E386D |
| | E386Q |
| | E386H |
| | E386N |
| | F443I |
| | F443L |
| | F443A |
| | F443V |
| | F443H |
| | F443Y |
| | F654A |
| | F654L |
| | F624A |
| | L656I |
| | L656E |
| | L656N |
| | V45Q + L656E |
| | V45L + T326S |
| | T326D + E386T |
| | F385Y + F654Y |
| (2) introduce mutations that can increase the cation-n stabilization of the carbocation intermediate, based on the structural and coevolution hotspots | F385Y |
| | F443Y |
| | F624W |
| | F624Y |
| | F654W |
| | F654Y |
| | L656F |
| | L656Y |
| | L656W |
| | F385Y + F654Y |
| | F385Y + F654Y + F443Y |
| (1) improve the Michaelis-Menten complex; and | W179A |
| | W179V |
| (3) open the catalytic cavity by mutating the residues that are only essential for the catalysis of the 5-ring native substrate, squalene | |
| (1) improve the Michaelis-Menten complex; and | T326E |
| | T326D |
| (4) mutate the residues that assist the last proton acceptor in order to facilitate product formation | T326S |
| | T326N |
| | T326C |
| | T326N + I278N |
| (1) improve the Michaelis-Menten complex; | I278A |
| (3) open the catalytic cavity by mutating the residues that are only essential for the catalysis of the 5-ring native substrate, squalene; and | I278V |
| | I278F |
| | I278Y |
| (4) mutate the residues that assist the last proton acceptor in order to facilitate product formation | I278N |
| (5) alter the active center | Q178H |
| | Q178E |
| | Q178D |
| | D397C |
| | D397C + D394V + V471C |
| (6) mutate residues responsible for the narrow constriction between the hydrophobic channel and the active center cavity | M184L |
| | M184V |
| | M184I |
| | M184C |
| | M184A |
| (7) and increase the QW motifs | R194Q |
| | M305W |
| | S321W |
| | S321F |
| | P412Q |
| | M345W |
| Other | W87L |
| | W87V* |
| | L335F |
| | S321F |
| | F460A |
| | F460H |
| | F460L |
| | Y658F |
| | W556A |
| | W556V* |
| | Q279V + Q54V |
| | D397C + D394V |
| | F385H + E386A |
| | F385E + E386A |
| | F385Y + F654Y + F443Y |

| | |
|---|---|
| *From the literature. | |

*SHC Mutant Enzyme Expression.* Wild-type and the GmSHC mutants of Table 6 were individually cloned into pET28a(+). These DNA constructs were transformed into BL21(DE3) *E. coli* and plated onto agar plates containing Kanamycin. These were incubated overnight at 37°C. A single bacterial colony was picked and used to inoculate 500 µL LB + Kanamycin in a 96-well plate. This plate was incubated overnight at 37°C with agitation. These primary cultures (10 µL) were used to inoculate 10 mL LB + Kanamycin in a 50 mL falcon tube, which was subsequently incubated at 37°C at 180 rpm for about 7 hours. Protein expression was then induced with the addition of 1 mM IPTG. The incubator temperature was lowered to 25°C and the cultures further incubated at 180 rpm overnight. The next day, the cultures were centrifuged at 4000 rpm for 10 minutes and the supernatant discarded. Cell pellets were exposed to 2 rounds of freeze/thawing before use in the reaction assay.

Cell pellet (1 µL) was spotted onto a nitrocellulose membrane and allowed to air dry for 30 minutes. The membrane was placed into 5% milk powder for 1 hour at room temperature with gentle agitation. The membrane was then rinsed with phosphate-buffered saline (PBS), 3x5 minutes. Anti-histidine antibody solution (1 in 10,000 dilution) was added and incubated at room temperature for 1 hour with shaking. The blot was subsequently washed in PBS, 3x5 minutes. Developing solution (6 mg diaminobenzidine (DAB) and 5 µL 30% H₂O₂ in 10 mL PBS) was added to the blot. Once developed, the developing solution was immediately removed and the blot rinsed with water.

The results of this analysis indicated that all constructs were expressed in BL21 (DE3) *E. coli* at 25°C following the addition of 1 mM IPTG. Following expression and processing of the enzymes, a dot blot was performed to assess if the introduction of specific mutations had altered the protein expression. Notably, the majority of the GmSHC mutants showed similar levels of expression to the wild-type GmSHC construct.

*SHC Mutant Screening Reactions.* Sodium citrate buffer pH 5.3 (equal volumes of 1 M sodium citrate, pH 4.9 and 0.1 M sodium citrate, pH 6.5) was prepared and 500 µL of this buffer was added to freeze-dried whole cells of each mutant (from 1L shake flask fermentation of *E. coli* transformed with desired mutant plasmid). The results were obtained with an enzyme loading of 5% (w/w). Subsequently, homofarnesol (50 mg/mL) was added to the buffer/enzyme mix. The reactions were incubated at 37°C with agitation for 18 hours. To stop the reaction and extract the products, a 2X volume of 3:2, heptane:isopropanol was added to each reaction. These were then incubated at 37°C for 30 minutes with agitation to mix thoroughly. The reactions were centrifuged for 10 minutes at 4000 rpm to pellet any cellular material. The upper organic layer was then removed and placed in a clean gas chromatography (GC) vial.

*GC Analysis Method.* A GC analytical method was used to detect each of the starting materials and products used in the screening reactions. Due to the volume of samples generated, a fast method was developed with a run time of only 4.5 minutes. The GC analysis conditions are presented in Table 7.

**TABLE 7**

| Component | | Condition | | |
|---|---|---|---|---|
| GC system | | Perkin Elmer Autosystem XL | | |
| Column | | Agilent HP-5 (30 m, 0.25 mm x 0.25µm) | | |
| Carrier gas | | Helium | | |
| Carrier pressure | | 30 psi | | |
| Oven program | | Rate/°C min⁻¹ | Temperature/°C | Hold/min |
| | | 0 | 200 | 1.5 |
| | | 45 | 225 | 0.5 |
| | Injection temperature | 270°C | | |
| | Detector | FID | | |
| | Detector temperature | 270°C | | |
| | Injection volume | 1 µL | | |
| | Syringe volume | 10 µL | | |
| | Data acquisition time | 4.5 minutes | | |

Analysis of the SHC mutant reaction samples following the addition of both 3 and 10 mg/mL homofarnesol indicated that a number of SHC mutants/derivatives exhibited improved activity compared to the wild-type enzyme. Mutants of particular interest are listed in Table 8.

**TABLE 8**

| Mutation | Average increase in ambrox production vs SHC WT (% peak area) |
|---|---|
| M184A | +21 |
| F624Y | +21 |
| V45L + T326S | +18.5 |
| M184L | +18.5 |
| E46H | +18 |
| Q54E | +18 |
| R194Q | +14.5 |
| F624A | +14 |
| Y658F | +14 |
| G623A | +14 |
| M184I | +13 |
| Q178E | +12.5 |
| E46Q | +12.5 |
| M184V | +11 |
| F460A | +6 |

The results demonstrated that many of the enzymes reached full consumption of the homofarnesol isomer used to generate the ambrox. Accordingly, further analysis was conducted to identify one or more optimal SHC enzymes. In particular, substrate loading was increased to 15 mg/mL and incubation was allowed to progress for a limited period of time (*i.e*., 4 hours and 20 hours). As shown in Table 9, there were multiple mutant enzymes which displayed higher activity than the wild-type SHC enzyme. In particular, the F624Y SHC mutant showed the highest activity after 4 hours, whereas the E46Q SHC mutant showed the highest activity after 20 hours. Notably, each the enzymes with mutations at position 184 (M184L, M184V, M184I and M184A), which were designed to affect the enzyme specificity by changing the hydrophobic channel that gives access to the active center, exhibited an increase in activity following a longer incubation period.

**TABLE 9**

| Mutation | 4 hours at 37°C | | 20 hours at 37°C | |
|---|---|---|---|---|
| | Average ambrox peak area % | Average difference (% peak area) * | Average ambrox peak area % | Average difference (% peak area) * |
| V45L + T326S | 48.5 | +11 | 68 | +20.5 |
| G623A | 36 | -1.5 | 74.5 | +27 |
| F624Y | 55 | +17.5 | 68.5 | +21 |
| F624A | 33.5 | -4 | 54.5 | +7 |
| F460A | 26.5 | -11 | 60.5 | +13 |
| E46Q | 45.5 | +8 | 82.5 | +35 |
| M184L | 40 | +2.5 | 68 | +20.5 |
| M184V | 40.5 | +3 | 69 | +21.5 |
| M184I | 47.5 | +10 | 80.5 | +33 |
| M184A | 34 | -3.5 | 66 | +18.5 |
| R194Q | 33.5 | -4 | 59 | +11.5 |
| Y658F | 29 | -8.5 | 51 | +3.5 |
| Q178E | 48 | +10.5 | 67.5 | +20 |
| E46H | 32.5 | -5 | 55.5 | +8 |
| Q54E | 27.5 | -10 | 66 | +18.5 |
| Wild-type | 37.5 | 0 | 47.5 | 0 |

| | | | | |
|---|---|---|---|---|
| *Average difference in ambrox production vs SHC wild-type. | | | | |

In addition to ambrox, mutants were also tested for sclareolide production from homofarnesic acid. This analysis indicated that the G623V, I278V, L335F and Q54E mutants exhibited an increase in sclareolide production compared to wild-type GmSHC (Table 10).

**TABLE 10**

| Mutation | Average increase in sclareolide production vs SHC WT (% peak area) |
|---|---|
| L656E | +0.2 |
| V45I | +0.3 |
| V45Q | +0.2 |
| G623A | +0.2 |
| G623V | +1.6 |
| I278V | +1.7 |
| E386Q | +0.2 |
| L335F | +1.6 |
| Q178E | +1 |
| E46H | +1 |
| Q54E | +1.4 |

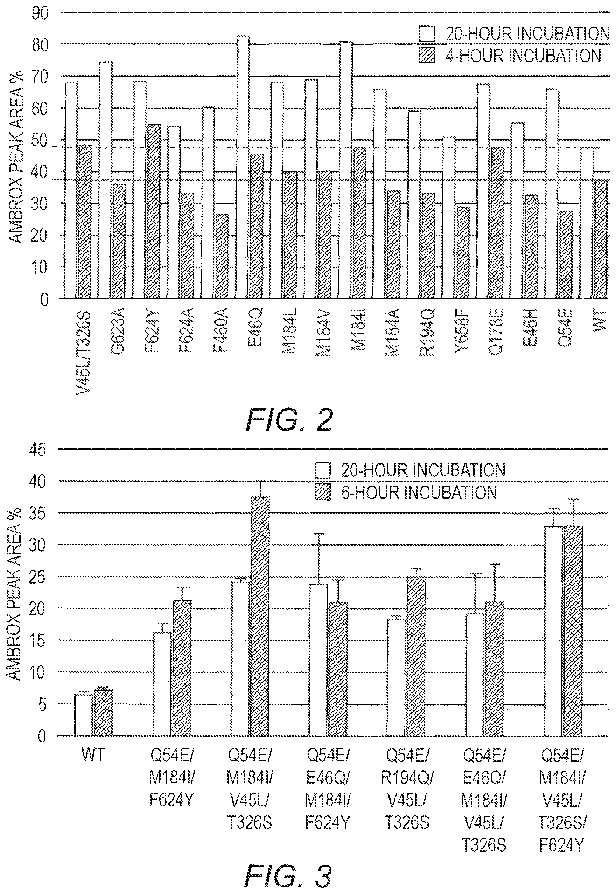

FIG. 2 shows that after the 4-hour incubation only seven mutants demonstrated higher ambrox production than the wild-type SHC (bars all above the dashed line), whereas 15 mutants exhibited higher activity following the 20-hour incubation (FIG. 2). In particular, the V45L + T326S, F624Y, E46Q, M184L, M184V, M184I and Q178E mutants demonstrated increased activity at both time points. In addition to these seven mutants, *in silico* and *in vitro* analyses indicated that the G623A, Q54E, R194Q and M184A mutants were also of interest. Accordingly, combination mutants are provided, which exhibit additive or synergistic effects to increase the activity of the GmSHC enzyme (Table 11).

**TABLE 11**

| Conjugated Mutants | | |
|---|---|---|
| E46Q + M184I | E46Q + M184A | E46Q + M184V |
| E46Q + M184I + G623A | E46Q + M184I + F624Y + G623A + V45L + T326S | E46Q + M184I + F624Y + G623A |
| M184I + Q178E | E46Q + F624Y | Q54E + M184I |
| M184V + Q178E | E46Q + G623A | E46Q + M184I + F624Y |
| E46Q + M184I + F624Y + R194Q + V45L + T326S + G623A | E46Q + M184I + R194Q | E46Q + M184I + V45L + T326S |
| E46Q + M184I + F624Y + R194Q | E46Q + M184I + F624Y + R194Q + V45L + T326S | E46Q + M184L |
| Q54E + R194Q | Q54E + E46Q | Q54E + M184I + F624Y |
| Q54E + M184I + V45L + T326S | Q54E + M184I + F624Y + T326S | Q54E + E46Q + M184I + F624Y |
| Q54E + R194Q + V45L + T326S | Q54E + E46Q + M184I + V45L + T326S | Q54E + M184I + V45L + T326S + F624Y |

When selected combination mutants were incubated with 50 mg/mL homofarnesol for 20 hours, it was found that each of the mutants exhibited an increase in activity compared to wild-type GmSHC (Table 12). Similar results were observed when the combination mutants were incubated with 50 mg/mL homofarnesol for 6 or 20 hours at 25% enzyme loading (FIG. 3).

**TABLE 12**

| Mutation | % Area Ambrox Product Peak |
|---|---|
| Wild-type | 18.25 |
| E46Q + M184I + V45L + T326S | 21.20 |
| Q54E + M184I | 21.86 |
| Q54E + R194Q | 21.39 |
| Q54E + E46Q | 20.02 |
| Q54E + M184I + F624Y | 35.10 |
| 6 | 28.96 |
| Q54E + M184I + V45L + T326S | |
| Q54E + M184I + F624Y + T326S | 21.57 |

### Example 6: Subsequent Rounds of Semi-Rational Mutagenesis (via Site-Saturation Library) and Random Mutagenesis (Error Prone PCR)

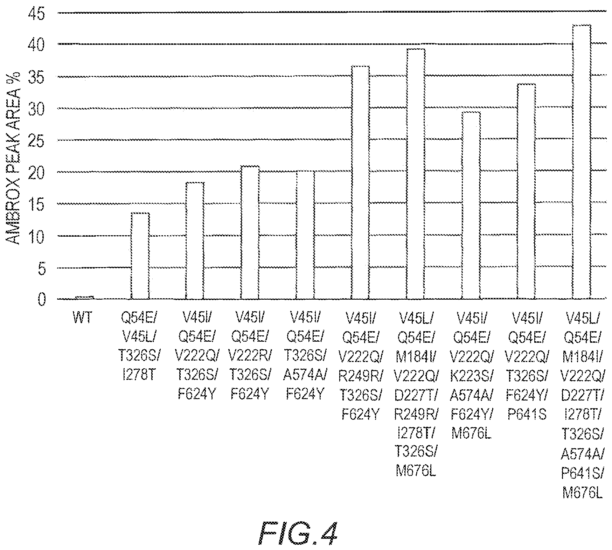

To further improve GmSHC activity, additional mutants were generated and screened for ambrox production as compared to wild-type (Table 13). GmSHC derivative screening assays included the use of 50 g/L homofarnesol with 2.5% or 5% enzyme loading for a 24-hour or 28-hour incubation period at 37°C. FIG. 4 illustrates the ambrox product peak % produced following the incubation of some SHC mutants when compared to wild-type.

**TABLE 13**

| Amino Acid Alteration | Average Difference (% Product Peak Area)* | Average Fold Improvement |
|---|---|---|
| WT | 0.3 | N/A |
| V45I/Q54E/V222Q/T326S/F624Y | 18.3 | 61.0 |
| V45I/Q54E/P166P/T326S/F624Y | 39.4 | 131.3 |
| V45I/Q54E/K223S/D227T/T326S/F624Y | 23.3 | 77.7 |
| V45L/Q54E/M184I/V222Q/D227T/I278T/ T326S/A574A/M676L | 17.3 | 57.7 |
| V45I/Q54E/K223S/T326S/A574A/F624Y | 21.6 | 72.0 |
| V45I/Q54E/T326S/A574A/F624Y | 20.0 | 66.7 |
| V45I/Q54E/D227T/T326S/F624Y/M677E | 28.6 | 95.3 |
| V45I/Q54E/V222Q/T326S/F624Y/M676L | 23.6 | 78.7 |
| V45I/Q54E/V222R/T326S/F624Y | 20.8 | 69.3 |
| V45I/Q54E/V222Q/T326S/F624Y/S682R | 27.2 | 90.7 |
| V45I/Q54E/V222Q/R249R/T326S/F624Y | 36.6 | 122 |
| V45L/Q54E/M184I/V222Q/D227T/R249R/ I278T/T326S/M676L | 39.2 | 130.7 |
| V45L/Q54E/M184I/V222Q/S242R/D227T/ I278T/T326S/M676L | 35.5 | 118.3 |
| V45I/Q54E/K223S/D227T/T326S/F624Y/ L640G | 21.8 | 72.6 |
| E46Q/Q54E/R194Q/V222Q/D227T/A574A/ F624Y/L640G | 18.6 | 62.0 |
| V45L/Q54E/M184I/V222Q/D227T/I278T/T326S/M676L/P641S | 35.3 | 117.7 |
| V45I/Q54E/V222Q/K223S/A574A/F624Y/ M676L | 29.3 | 97.7 |
| V45L/Q54E/M184I/V222Q/D227T/I278T/ T326S/A574A/M676L /M677E | 26.15 | 87.2 |
| V45I/Q54E/V222Q/T326S/F624Y/P641S | 33.7 | 112.3 |
| V45L/Q54E/M184I/V222Q/D227T/I278T/ T326S/A574A/P641S/M676L | 42.7 | 142.3 |
| V45I/Q54E/V222R/T326S/F624Y/S682R | 20.2 | 67.3 |
| V45L/Q54E/M184I/V222Q/D227T/I278T/ T326S/R504C/A574A/P641S/M676L | 24 | 80.0 |
| V45L/Q54E/M184I/V222Q/D227T/R249R/ I278T/T326S/P641S/M676L | 24.5 | 81.7 |
| V45I/Q54E/M184I/V222Q/D227T/R249R/ I278T/T326S/R504C/M676L | 19 | 63.3 |
| V45L/Q54E/M184I/V222Q/D227T/I278T/ T326S/A574A/P641S/M676L/S682R | 18.8 | 62.7 |
| V45L/Q54E/M184I/V222R/S242R/D227T/ I278T/T326S/M676L | 28.9 | 96.3 |
| V45L/Q54E/M184I/V222R/D227T/I278T/ T326S/A574A/P641S/M676L | 25.6 | 85.3 |
| Q54E/V45L/T326S/I278T | **13.4** | 34.3 |

| | | |
|---|---|---|
| * Average difference in Ambrox production vs SHC-wild type enzyme | | |

It was observed that a V45I/Q54E/V222Q/T326S/F624Y GmSHC derivative, and a V45I/Q54E/K223S/D227T/T326S/F624Y GmSHC derivative showed good conversion of homofarnesol to ambrox, which does not plateau over time. At 100 mg/mL homofarnesol with 5% enzyme loading, the V45I/Q54E/K223S/D227T/T326S/F624Y GmSHC derivative performed better than fermentation with a Q54E/V45L/T326S/I278T GmSCH derivative, which had the best performance to date. In addition, V45I/Q54E/D227T/T326S/F624Y/M677E GmSCH derivative; V45I/Q54E/D227T/T326S/F624Y/A574A GmSCH derivative; and V45L/Q54E/M184I/V222Q/D227T/I278T/T326S/M676L/A574A GmSCH derivative all showed good ambrox production and higher conversions than seen previously. Moreover, these mutants exhibited good growth, expression and activity.

Introducing V222Q showed impressive enhancement in conversion of homofarnesol to ambrox when introduced into the 45L/Q54E/M184I/I278T/T326S and V45I/Q54E/T326S/F624Y GmSHC derivatives and V222R improved the conversion of homofarnesol to ambrox when introduced into the V45I/Q54E/V222Q/T326S/F624Y parental template. In addition, the alteration of V222Q to V222R in a Q54E/M184I/45L/T326S/I278T/D227T/V222Q/M767L/S242R parental template appeared to speed up the initial conversion of homofarnesol.

Introduction of A574A showed good improvements in conversion of homofarnesol to ambrox when introduced into the 45L/Q54E/M184I/V222Q/D227T/I278T/T326S/M767L; 45L/Q54E/M184I/V222Q/D227T/I278T/T326S/F654T; V45I/Q54E/ K223S/T326S/F624Y; and V45I/Q54E/D227T/T326S/F624Y GmSHC derivatives.

Introduction of L640G and M676L showed good improvements in conversion of homofarnesol to ambrox when introduced into the E46Q/Q54E/R194Q/V222Q/ D227T/A574A/F624Y; V45I/Q54E/V222Q/T326S/F624Y; and V45I/Q54E/K223S/D227T/T326S/F624Y GmSHC derivatives. Similarly, introduction of P641S into V45L/Q54E/M184I/V222Q/D227T/I278T/T326S/M676L improved homofarnesol to ambrox of this parental template. Positions 640 and 641 are located in a loop and mutation of either of these residues increases the flexibility of this loop. Given that the loop is near the narrow constriction of the channel, which is responsible for substrate recognition, adding flexibility was expected to have an indirect effect on the substrate recognition. However, the results of this analysis indicated that mutants possessing L640G or P641S amino acid mutations did not appear to be able to utilize isomers of homofarnesol other than the E,E isomer.

Introduction of P166P, S682R and M676L into the parental templates V45I/Q54E/T326S/F624Y; V45I/Q54E/V222Q/T326S/F624Y; and V45I/Q54E/K223S/T326S/A574A/F624Y GmSHC derivatives improved homofarnesol to ambrox conversion. However, S242R appeared to be detrimental to enzyme activity when introduced to the V45I/Q54E/V222Q/T326S/F624Y GmSHC derivative.

R249R was identified in an error prone library. Introduction of this silent mutation in Q54E/M184I/V45L/T326S/I278T/D227T/V222Q/M676L/P641S and Q54E/V45I/T326S/F624Y/V222R GmSHC derivatives enhanced homofarnesol to ambrox conversion compared to the parental GmSHC derivatives.

The mutation of M677 and M80 to Glu in Q54E/M184I/V45L/T326S/I278T/D227T/V222Q/M676L/A574A appeared to stabilize the enzyme and improved homofarnesol conversion.

Although the Q54E/M184I/V45L/T326S/I278T/D227T/ V222Q/M676L/P641S/A574A/R504C GmSHC derivative showed promising homofarnesol conversion, the introduction of R504C did not appear to improve the already impressive conversion observed by the parental template containing the Q54E/M184I/V45L/T326S/I278T/D227T/V222Q/M676L/P641S/A574A mutations.

GmSHC derivatives including the R249R insertion and S682R substitution showed an uplift when compared to the respective parental templates including the following mutations V45L/Q54E/M184I/V222Q/D227T/I278T/T326S/R504C/ A574A/P641S/M676L and V45L/Q54E/M184I/V222Q/D227T/I278T/ T326S/A574A/P641S/M676L.

### Example 7: Summary of GmSHC Derivatives

SHCs are integral monotopic membrane proteins that adopt a dimeric 3D arrangement. Each monomer is characterized by QW motifs that tightly connect numerous α-helices building up two highly stable α/α-barrels domains (Wendt et al. (1999) J. Mol. Biol. 286:175-87). The active center cavity is buried within the two α/α-barrels domains and its access is possible through an inner hydrophobic channel, which it is suggested to be the membrane-immersed region of the enzyme (Lenhart, et al. (2002) Chem. Biol. 9:639-45). The channel and the active center cavity are separated by a narrow constriction which is responsible for substrate recognition (Lenhart, et al. (2002) Chem. Biol. 9:639-45). For GmSHC, those residues correspond to Phe176, Met184, Phe457 and Cys458. The residues that constitute the conserved DXDD motif (Wendt et al. (1999) J. Mol. Biol. 286:175-87), are found at the top of the activity center cavity. One of those residues is Asp396, the first proton donor, which initiates cyclization of homofarnesol by donating a proton to the double bond C2=C3. The DXDD motif is followed by tryptophan, tyrosine and phenylalanine residues that are responsible for stabilizing the cationic intermediates by strong cation-n interactions (Dougherty

(1996) Science 271:163-8). On the bottom of the cavity is a negatively charged residue, the last proton acceptor, which receives a proton from the hydroxyl group thereby resulting in closure of the third ring and formation of ambrox.

To improve the conversion of homofarnesol to ambrox, GmSHC was mutated at one or more of the residues at position 45, 46, 54, 178, 184, 194, 247, 278, position 326, 386, 335, 460, 623 and 624 of SEQ ID NO:2.

Positions 45 and 326. Accordinct to the GmSHC homology model and molecular docking calculations, residues V45 and T326 are placed near the substrate hydroxyl group. GmSHC position 45 is mutated to glutamine, leucine or isoleucine and position 326 to serine in order to increase the intermolecular interactions with the substrate. The combination of both these mutations (V45L + T326S) showed a 1.4-fold increase in ambrox production after a 20-hour incubation with 15 g/L homofarnesol.

*Positions 46, 54* and *386.* According to GmSHC homology model and molecular docking calculations, residues E46 or E386 function as a last proton acceptor, receiving a proton from the substrate hydroxyl group. A structural alignment between AaSHC (Reinhert, et al. (2004) Chem. Biol. 11:121-6) and the GmSHC homology model indicates that Q54 of GmSHC is superimposed with residue E45 of AaSHC, which is the last proton acceptor of this enzyme (Dang & Prestwich (2000) Chem. Biol. 7:643-9). Therefore, residue 54 of GmSHC was mutated to glutamate to incorporate a last proton acceptor at this position, without having a negative impact on the charge network associated with the conserved DXDD motif. Residue 46 was mutated to glutamine, alanine or histidine, while residue 386 was mutated to glutamine to change the last proton acceptor position. When compared to the wild-type enzyme, mutants with mutations at position E386 had no effect on enzyme activity. However, mutations at positions E46 and Q54 both showed an increase in conversion of homofarnesol to ambrox. After a 20-hour incubation of 15 g/L homofarnesol, the E46Q and E46H mutants respectively exhibited a 1.8-fold and 1.2-fold improvement in activity, whereas the Q54E mutant exhibited a 1.4-fold improvement in activity compared to the wild-type enzyme.

*Position* 178. A structural alignment between the GmSHC homology model and the homologous human lanosterol synthase (Thoma, et al. (2004) Nature 432:118-22) indicates that Q178 of GmSHC is superimposed with residue H232 of the human lanosterol synthase, which is the last proton acceptor of this enzyme. Therefore, residue 178 of GmSHC was mutated to glutamate to incorporate a last proton acceptor at this position. The introduction of this mutation increased conversion of homofarnesol to ambrox by 1.4-fold compared to the wild-type enzyme.

*Position* 184. Residue M184 is placed in narrow constriction, which is responsible for substrate recognition (Lenhart, et al. (2002) Chem. Biol. 9:639-45). Thus, to alter the substrate recognition, M184 of GmSHC was mutated to non-polar amino acids, i.e., Leucine, Isoleucine, Valine and Alanine. By mutating this position, any methionine oxidation phenomenon is also prevented, which could negatively affect substrate recognition. It was observed that mutation of M184 to any one of Leu, Ile, Val or Ala resulted in an increase in activity. Notably, the M184I mutant exhibited the largest increase, with a 1.7-fold improvement in conversion of homofarnesol to ambrox when screened at the low substrate loading.

*Position 194.* QW motifs firmly connect the α-helices contributing to the build-up of two α/α-barrels. These highly stable α/α-barrels protect the *enzyme* against the energy release associated with the highly exergonic catalyzed reaction. According to the homology model of GmSHC, residue R194 is placed near residue W152. Therefore, residues 194 was mutated to glutamine in order to introduce a new QW motif with W152, thereby increasing the structural stability of the enzyme. Experimentally, this mutation showed improvements in conversion with a 1.3-fold improvement in conversion of homofarnesol to ambrox at low substrate loading.

*Position 247.* According to the GmSHC homology model, residue P247 is placed in a loop at the channel entrance, which is suggested to be the membrane-immersed region of the enzyme. Residue P247 was mutated to a non-proline residue to change the dynamics of the channel in this region. When tested in the combination mutant, V45L + T326S + M184I + R194Q, a 2.7-fold improvement in conversion was observed when incubated with 50 g/L homofarnesol at 40% enzyme loading.

*Position 278.* According to the GmSHC homology model and the molecular docking calculations, residue I278 is placed right below the substrate hydroxyl group. When residue I278 is mutated to valine, the molecular docking calculations indicate that the substrate arrangement within the active center improves by placing the substrate C2=C3 double bond closer to D396, the first proton donor. Mutation of residue I278 did not show any improvements in homofarnesol conversion; however, when the enzyme was incubated with homofarnesic acid, the conversion of acid to sclareolide showed a 2-fold improvement when compared to the wild type enzyme when tested at 10 g/L substrate loading and 100% enzyme loading. It was subsequently discovered that the introduction of threonine at this position instead of valine was extremely beneficial.

*Position 335.* According to the GmSHC homology model and the molecular docking calculations, residue L335 is placed near residue D396, the first proton donor, and when mutated to phenylalanine it can introduce a strong cation-n interaction with the substrate cationic intermediate. Mutations at this position provided a 1.8-fold improvement with regards to the conversion of homofarnesic acid to sclareolide.

*Position 460.* According to the GmSHC homology model, residue F460 of GmSHC is a residue in the active center cavity next to the narrow constriction that is responsible for substrate recognition (Lenhart, et al. (2002) Chem. Biol. 9:639-45). Residue F460 was mutated to alanine to increase the access of the substrate to the active center cavity. As a result, this mutation showed a 1.3-fold improvement when reacted with homofarnesol at 15 g/L substrate loading.

*Position 623.* According to the GmSHC homology model and the molecular docking calculations, residue G623 is positioned closed to hydroxyl group of the substrate. Accordingly, residue G623 was mutated to alanine or valine to increase the intermolecular interactions with the substrate. While the G623A mutant showed a 1.6-fold improvement in ambrox production in the presence of 15 g/L homofarnesol, the G623V mutant exhibited a 1.9-fold increase in the conversion of homofarnesic acid to sclareolide.

*Position 624.* According to the GmSHC homology model and the molecular docking calculations, residue F624 establishes a strong cation-π interaction, stabilizing the cationic intermediate. Therefore, residue F624 was mutated to tyrosine or tryptophan to introduce an even stronger cation-π interaction with the substrate cationic intermediate. When this position was altered to tryptophan, a 1.45-fold improvement in the production of ambrox from homofarnesol was obtained.

*Position 222.* Each SHC monomer is characterized by QW motifs that tightly connect numerous α-helices building up two highly stable α/α-barrels domains (Wendt, et al. (1999) J. Mol. Biol. 286(1):175-87). Mutant V222Q was designed to establish a new QW motif with W229, which further increases the structural stability of the enzyme. In a sequence alignment of 1000 homologous enzymes of *Gluconobacter morbifer* SHC (GmSHC)*,* the consensus residue for position 222 is arginine. Consensus residues, like V222R, are typically associated with a higher structural stability (Steipe, et al. (1994) J. Mol. Biol. 240(3):188-92). Therefore, mutation of V222 to Q or R was expected to improve activity.

*Position 223.* K223S directly interacts with residue F460 of the narrow constriction that separates the channel from the active center and is responsible for substrate recognition (Lenhart, et al. (2002) Chem. Biol. 9(5):639-45). Mutant K223S can change the dynamics of the loop where it is placed and indirectly affects the narrow constriction responsible for substrate recognition.

*Position 226.* The negative charges of E225, E226 and D227 side chains are not contra-balanced by any positively charged residues in the vicinity. This motif is not observed in any of the 1000 homologous enzymes of *Gluconobacter morbifer* SHC (GmSHC) analyzed. Mutant E226V decreased the repulsion effect of having three consecutive negatively charged residues without adding any steric penalty thereby increasing enzyme stability.

*Position 227.* D227T can establish dipole-dipole interactions with the side chain and main chain of T453. This mutation also decreases the repulsion associated with having three consecutive negatively charged residues (E225, E226 and D227). Both effects contribute to increase enzyme stability.

*Position 242.* Residue S242 is placed on the alpha-helix responsible for the enzyme: membrane interaction. See Gustafsson, et al. (2017) ACS Omega 2(11):8495-8506.

*Position 249.* This residue interacts with the cell membrane.

*Position 504.* Position 504 is placed in a loop. Mutant R504C introduces an ion-dipole with E503 side chain and a dipole-dipole interaction with N505 side chain, which increases the stability of the loop and contributes to increase the overall structural stability of the enzyme.

*Position 640.* Position L640 is placed in a loop. Mutant L640G increases the flexibility of this loop, which is near the narrow constriction of the channel that is responsible for substrate recognition (Lenhart, et al. (2002) Chem. Biol. 9(5):639-45). Adding flexibility in this loop may have an indirect effect on the substrate recognition.

*Position 641.* Position P641 is placed in a loop. Mutation of this residue contributes to a local rearrangement of the loop as it removes a rigid proline and introduces a more flexible residue. This was expected to have an indirect effect on the substrate recognition since position 641 is close to the narrow constriction of the channel that is responsible for substrate recognition (Lenhart, et al. (2002) Chem. Biol. 9(5):639-45) . Moreover, P641S introduces the consensus residue in this position. Consensus residues are typically associated with a higher structural stability (Steipe, et al. (1994) J. Mol. Biol. 240(3):188-92).

*Position 676.* Residue M676 is in the C-terminal region of the enzyme and is exposed to the solvent. An enzyme with a M676L substitution prevents methionine oxidation triggered by a long exposure to the solvent.

*Position 677.* Residue M677 is in the C-terminal region of the enzyme and is exposed to the solvent. An enzyme with a M676L substitution prevents methionine oxidation triggered by a long exposure to the solvent. Additionally, M677E introduces the consensus residue in this position. Consensus residues are typically associated with a higher structural stability.

*Position 682.* Residue S682 at the end of C-terminal region of the enzyme and is exposed to the solvent. The C-terminus is composed of a considerably high number of charged residues (D680, E679, K678, R675, R674, and R672). S682R adds one more charged residue to the C-terminus, which was observed to be beneficial for the activity of the enzyme.

## Claims

1. A recombinant vector comprising a nucleic acid molecule encoding a Squalene Hopene Cyclase (SHC) polypeptide having at least 90% sequence identity to SEQ ID NO:2 and including amino acid alterations, relative to SEQ ID NO:2, at positions 45, 54, 184 222, 227, 278, 326, 574, 641 and 676, wherein the amino acid alterations comprise V45L, Q54E, M184I, V222R, D227T, I278T, T326S, A574A, P641S and M676L,
wherein the amino acid alterations enhance the activity of the SHC polypeptide for converting homofarnesol to ambrox as compared to wild-type SHC, the wild-type SHC having the amino acid sequence of SEQ ID NO: 2.

2. A recombinant host cell comprising the recombinant vector of claim 1.

3. A recombinant Squalene Hopene Cyclase (SHC) polypeptide having at least 90% sequence identity to SEQ ID NO:2 and including amino acid alterations, relative to SEQ ID NO:2, at positions 45, 54, 184 222, 227, 278, 326, 574, 641 and 676, wherein the amino acid alterations comprise V45L, Q54E, M184I, V222R, D227T, I278T, T326S, A574A, P641S and M676L,
wherein the amino acid alterations enhance the activity of the SHC polypeptide for converting homofarnesol to ambrox as compared to wild-type SHC, the wild-type SHC having the amino acid sequence of SEQ ID NO: 2.

4. A method for producing ambroxan comprising
(a) providing homofarnesol to the recombinant host cell of claim 2, and
(b) collecting ambroxan produced by the host cell.

5. The method of claim 4, wherein the homofarnesol comprises (3E,7E) homofarnesol.

## Patentansprüche

1. Rekombinanter Vektor, umfassend ein Nukleinsäuremolekül, das ein Squalen-Hopen-Cyclase(SHC)-Polypeptid codiert, das mindestens 90 % Sequenzidentität mit SEQ ID NO: 2 aufweist und Aminosäureänderungen relativ zu SEQ ID NO: 2 an den Positionen 45, 54, 184, 222, 227, 278, 326, 574, 641 und 676 enthält, wobei die Aminosäureänderungen V45L, Q54E, M184I, V222R, D227T, I278T, T326S, A574A, P641S und M676L umfassen,
wobei die Aminosäureänderungen die Aktivität des SHC-Polypeptids zum Umwandeln von Homofarnesol zu Ambrox im Vergleich zu Wildtyp-SHC verbessern, wobei die Wildtyp-SHC die Aminosäuresequenz unter SEQ ID NO: 2 aufweist.

2. Rekombinante Wirtszelle, umfassend den rekombinanten Vektor nach Anspruch 1.

3. Rekombinantes Squalen-Hopen-Cyclase(SHC)-Polypeptid, das mindestens 90 % Sequenzidentität mit SEQ ID NO: 2 aufweist und Aminosäureänderungen relativ zu SEQ ID NO: 2 an den Positionen 45, 54, 184, 222, 227, 278, 326, 574, 641 und 676 enthält, wobei die Aminosäureänderungen V45L, Q54E, M184I, V222R, D227T, I278T, T326S, A574A, P641S und M676L umfassen,
wobei die Aminosäureänderungen die Aktivität des SHC-Polypeptids zum Umwandeln von Homofarnesol zu Ambrox im Vergleich zu Wildtyp-SHC verbessern, wobei die Wildtyp-SHC die Aminosäuresequenz unter SEQ ID NO: 2 aufweist.

4. Verfahren zur Erzeugung von Ambroxan, umfassend
(a) Bereitstellen von Homofarnesol für die rekombinante Wirtszelle nach Anspruch 2 und
(b) Sammeln von Ambroxan, das von der Wirtszelle erzeugt wurde.

5. Verfahren nach Anspruch 4, wobei das Homofarnesol (3E, 7E)-Homofarnesol umfasst.

## Revendications

1. Vecteur recombinant comprenant une molécule d'acide nucléique codant pour un polypeptide de squalène hopène cyclase (SHC) ayant une identité de séquence d'au moins 90 % avec la SEQ ID NO : 2 et comprenant des modifications d'acides aminés, par rapport à la SEQ ID NO : 2, aux positions 45, 54, 184, 222, 227, 278, 326, 574, 641 et 676, dans lequel les modifications d'acides aminés comprennent V45L, Q54E, M184I, V222R, D227T, I278T, T326S, A574A, P641S et M676L,
dans lequel les modifications d'acides aminés augmentent l'activité du polypeptide SHC pour convertir l'homofarnésol en ambrox par comparaison avec le SHC de type sauvage, le SHC de type sauvage ayant la séquence d'acides aminés de la SEQ ID NO : 2.

2. Cellule hôte recombinante comprenant le vecteur recombinant de la revendication 1.

3. Polypeptide de squalène hopène cyclase (SHC) recombinant ayant une identité de séquence d'au moins 90 % avec la SEQ ID NO : 2 et comprenant des modifications d'acides aminés, par rapport à la SEQ ID NO : 2, aux positions 45, 54, 184, 222, 227, 278, 326, 574, 641 et 676, dans lequel les modifications d'acides aminés comprennent V45L, Q54E, M184I, V222R, D227T, I278T, T326S, A574A, P641S et M676L,
dans lequel les modifications d'acides aminés augmentent l'activité du polypeptide SHC pour convertir l'homofarnésol en ambrox par comparaison avec le SHC de type sauvage, le SHC de type sauvage ayant la séquence d'acides aminés de la SEQ ID NO : 2.

4. Procédé de production d'ambroxane comprenant
(a) la fourniture d'homofarnésol à la cellule hôte recombinante de la revendication 2, et
(b) la collecte d'ambroxane produit par la cellule hôte.

5. Procédé selon la revendication 4, dans lequel l'homofarnésol comprend le (3E, 7E) homofarnésol.
